(19)

Europäisches Patentamt
European Patent Office
Office européen des brevets

(11) **EP 4 368 240 A1**

(12) **EUROPEAN PATENT APPLICATION**

(43) Date of publication:
**15.05.2024 Bulletin 2024/20**

(21) Application number: **22386079.2**

(22) Date of filing: **11.11.2022**

(51) International Patent Classification (IPC):
***A61N 1/05*** (2006.01)       ***A61N 1/36*** (2006.01)
***A61N 1/378*** (2006.01)

(52) Cooperative Patent Classification (CPC):
**A61N 1/0556; A61N 1/36003; A61N 1/36017;
A61N 1/36062; A61N 1/36139;** A61N 1/3787

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Cambridge Enterprise, Ltd.
Cambridge Cambridgeshire CB2 1TN (GB)**

(72) Inventor: **The designation of the inventor has not
yet been filed**

(74) Representative: **J A Kemp LLP
80 Turnmill Street
London EC1M 5QU (GB)**

(54) **MEDICAL DEVICES, SYSTEM AND METHODS OF TREATMENT**

(57)     The present invention provides a system for treating a patient having a spinal cord injury, the system including: a first flexible implantable device and having a first plurality of electrodes; a second flexible implantable device and having a second plurality of electrodes; and a controller communicatively coupled to the electrodes of the first and second implantable devices and arranged to receive inputs from the electrodes of the first implantable device, process said inputs to produce stimulation signals and send said stimulation signals to the electrodes of the second implantable device. The devices may be configured for implantation at a number of sites in the patient's body, including the brain, spinal cord, nerves or limbs. The controller may be arranged to: process the inputs by decoding the inputs to determine an intended stimulation of nerves of the patient rostral of the position of the first device on the spinal cord; and produce stimulation signals that, when applied to the patient's spinal cord by the second device, produce said intended stimulation of the nerves of the patient. A method of treating spinal cord injury is also provided.

Figure 1

**Description**

The present invention relates to medical devices, systems and methods of treatment. The invention is of particular, but not exclusive, relevance to implantable devices, for example for treating spinal cord injury.

Spinal cord injury (SCI) is a disabling neurological condition that can lead to irreversible loss of sensory, motor and autonomic functions. Spinal cord injury (SCI) affects over 1.5 million people globally, including between 250,000 and 500,000 additional people every year. SCI occurs when the neuronal tracts of the spinal cord are damaged.

SCI also leads to a socioeconomic burden for the individual, their families and the healthcare system in supporting them.

Recent advances in targeted spinal cord stimulation (SCS) have enabled the restoration of assisted walking in patients paralyzed by SCI [1-3]. These pilot studies rely on the open-loop initiation of a stimulation algorithm or external, closed-loop triggering via inertial measurement units. Alternatively, closed-loop SCS has also been used, by decoding internal neural signals from the brain cortex [4]. These approaches aim to restore motor function after SCI and focus on dorsal stimulation of the spinal cord by using commercially available SCS devices, which primarily target chronic pain. The mechanism has been postulated to be indirect activation of motor neurons through proprioceptive circuits located dorsally [5].

Previous methods of spinal cord recording have employed the use of penetrating electrodes [9, 10], with advances in electrode fabrication allowing the implantation of multiple electrode arrays to increase the area of simultaneous neural recording [25, 26]. Penetrating electrodes, however, can lead to spinal cord injury along the electrode insertion tract and translating to chronic applications faces issue of wire breakage [25] and elastic modulus mismatches leading to chronic neural injury [27].

The present invention aims to solve one or more of the above problems.

Aspects of the present invention aim to provide systems and methods which can effectively treat or overcome the effects of spinal cord injury and restore a level of motion to a patient suffering from SCI, preferably without invasive surgery, or with minimally-invasive surgery.

Aspects of the present invention also aim to provide systems and methods of treating spinal cord injury using electrical sensing and stimulation.

Aspects of the present invention provide systems and methods for treating a patient having a spinal cord injury which use implanted electrodes to detect signals from the patient's brain or nervous system, process those signals and produce and deliver stimulation signals back to the patient's nervous system.

A first aspect of the present invention provides a system for treating a patient having a spinal cord injury, the system including: a first flexible implantable device and having a first plurality of electrodes; a second flexible implantable device and having a second plurality of electrodes; and a controller communicatively coupled to the electrodes of the first and second implantable devices and arranged to receive inputs from the electrodes of the first implantable device, process said inputs to produce stimulation signals and send said stimulation signals to the electrodes of the second implantable device.

The devices may be configured to be implanted in a range of locations within the patient. Without limitation, the devices may be configured for implantation on, around or adjacent to a nerve, a muscle (for example in a limb of the patient), the spinal cord or the brain. In particular embodiments, the first device may be implantable in the brain or adjacent to the spinal cord or nerve of a patient, for example rostrally (i.e. closer to the brain) in relation to a site of SCI. In particular embodiments the second device may be implantable adjacent to the spinal cord or nerve, or in a limb of a patient caudally (i.e. further from the brain) than a site of SCI.

In some embodiments one or both of the first and second implantable devices are configured to conform to the spinal cord or a nerve of the patient when implanted in the patient. Implantation to a site adjacent to the spinal cord or a nerve of the patient allows for close coupling between the electrodes and the nerves of the patient, both for detecting signals from the nerves and for stimulating the nerves. Configuring the device to conform to the spinal cord or nerve can further improve that coupling.

To improve the conforming of the devices, the devices of some embodiments have a gel layer arranged on a surface of the device opposed to a surface of the device having the plurality of electrodes (i.e. if the electrodes are on the "front" the device, then the gel layer is provided on the "back" of the device).

Preferably the gel layer is expandable or swellable, for example by being hydrophilic and absorbing water from tissue surrounding the implantation site. The gel layer can thus expand once implanted and fill the space surrounding the implantation site. This results in the device applying a light (i.e. non-compressive) pressure to the spinal cord when placed subdurally (or to a nerve) which can hold the device in the desired position as well as providing for close contact between the electrodes and the spinal cord or nerve.

In certain embodiments either or both of the first and second implantable devices are configured so that they are biased into a cylindrical configuration. In this manner the devices can be made self-rolling or self-cuffing so that they naturally conform to the spinal cord, nerve or other implantation site so as to hold the device in the desired position and/or provide for close contact between the electrode and the spinal cord or nerve. Various mechanical approaches can be used to create such a bias. For example, in some embodiments the devices may be formed from a plurality of polymer layers and a stress mismatch created between the polymer layers so as to cause a natural bias towards rolling up. The stress mismatch may be achieved by making the different polymer layers (e.g. top and bottom layers) different thicknesses, by asymmetrical thermal annealing, or by engineering mechanical features into the device, for example by etching.

In certain embodiments either or both of the first and second implantable devices are configured to substantially encircle the spinal cord or nerve of the patient when implanted in the patient and preferably completely encircle the spinal cord or nerve. By encircling the spinal cord or nerve, the device can closely conform to the spinal cord or nerve and good coupling between the electrodes and the nerves can be achieved. By having the first device encircle the spinal cord, the controller can use the spatial (i.e. circumferential) arrangement of the electrodes to interpret and identify the signals coming from all of the nerves in the spinal cord to determine the signals which are to be acted on. Similarly, by having the second device encircle the spinal cord, the controller can stimulate selected electrodes based on their position to cause stimulation of specific nerves within the spinal cord.

In certain embodiments the first and second implantable devices each have a distal portion and each distal portion contains the respective plurality of electrodes, further wherein the distal portions of the first and second implantable devices are identical. The other portions of the devices may also be identical. Thus a simplified system which makes use of identical devices for detection and stimulation can be used.

Preferably the controller is arranged to: process the inputs by decoding the inputs to determine an intended stimulation of nerves of the patient rostral of the position of the first device on the spinal cord; and produce stimulation signals that, when applied to the patient's spinal cord by the second device, produce said intended stimulation of the nerves of the patient. Thus the system can effectively bypass a SCI site by picking up the nerve signals above (i.e. on the brain side of) the site and stimulating nerves below the site to effectively re-apply the desired stimulation to the nerves for onward stimulation of muscles.

Preferably the controller is arranged to perform the steps of receiving, processing and sending substantially in real-time. For these purposes "real-time" is to be understood to mean with a similar degree of latency to that which would be physiologically present in an uninjured spinal cord.

In certain embodiments the controller is arranged to process said inputs by integrating the inputs in both the time and spatial domains. The controller may also be further arranged to identify unique topographical signatures from the integrated inputs to identify the dominant spinal cord tract which was activated. Integration in both time and spatial domains and/or the identification of topographical signatures from the inputs can allow processing of the complex signals obtained from, for example, the plurality of nerves in the spinal cord, to identify signals of interest and to determine the appropriate stimulation outputs corresponding to those signals.

The controller may be coupled to the electrodes of the devices through wired or wireless connections. Wireless connections may use any known protocols. Wired connections can improve (i.e. reduce) latency and can reduce the complexity of the individual devices, but can also require more complicated surgical planning and implantation of the wires.

The controller itself may be implantable, which is particularly beneficial where the coupling to the electrodes is wired.

The controller may be configured to wirelessly receive electrical power from a separate power source. In such a configuration, the controller and devices can be implanted in the patient, but it is not necessary to implant a power source. Using an external power source means that further surgery is not necessary to replace the power source.

The first plurality of electrodes and/or the second plurality of electrodes may be arranged in a regularly-spaced array. Different configurations of the array may be chosen. For example the electrodes may be arranged in a plurality of lines (for example extending longitudinally along the distal end of the device such that the line of electrodes can encircle the nerve or spinal cord). Electrodes in adjacent lines may be

Preferably each of the implantable devices has an overall thickness of less than $100\mu m$. More preferably each of the devices has an overall thickness of less than $50\mu m$ and, in some embodiments, an overall thickness of less than $10\mu m$.

Preferably the devices are configured for implantation and/or explantation through an incision of no more than 1 cm.

The system of the above aspect may include some, all or none of the above-described optional and preferred features in any combination.

A further aspect of the present invention provides a flexible implantable device having a plurality of electrodes. The device of this aspect is preferably one of the devices which is used in the system of the above aspect and may have some, all or none of the above-described preferred and optional features of such a device.

According to a further aspect of the invention, there is provided a method of treating a human or animal body, the method comprising implanting a medical system or device according to the above-described aspects.

A further aspect of the present invention provides a method of treating a patient having a spinal cord injury, the method including the steps of: percutaneously implanting a first plurality of electrodes to a position upstream of the location of the spinal cord injury; percutaneously implanting a second plurality of electrodes to a position downstream of the location of the spinal cord injury; processing signals from one or more of the first plurality of electrodes to determine an intended stimulation of nerves of the patient downstream of the position of the spinal cord injury; producing stimulation signals that, when applied to the patient's nerves through one or more of the second plurality of electrodes, produce said intended stimulation of the nerves of the patient.

The electrodes may be implanted in a range of locations within the patient. Without limitation, the implantation may be: around or adjacent to a nerve, a muscle (for example in a limb of the patient), the spinal cord or the brain. In particular embodiments, the first plurality of electrodes may be implanted in the brain or adjacent to the spinal cord or nerve of a patient, for example rostrally (i.e. closer to the brain) in relation to a site of SCI. In particular embodiments the second plurality of electrodes may be implanted adjacent to the spinal cord or nerve, or in a limb of a patient caudally (i.e. further from the brain) than a site of SCI.

In certain embodiments either or both of the first plurality of electrodes and the second plurality of electrodes are implanted such that they substantially encircle the spinal cord or a nerve of the patient, and preferably completely encircle the spinal cord or nerve. By encircling the spinal cord or nerve, the electrodes can closely conform to the spinal cord or nerve and good coupling between the electrodes and the nerves can be achieved. By having the first plurality of electrodes implanted so as to encircle the spinal cord, the spatial (i.e. circumferential) arrangement of the electrodes can be used in the step of processing to interpret and identify the signals coming from all of the nerves in the spinal cord to determine the signals which are to be acted on. Similarly, by having the second plurality of electrodes encircle the spinal cord, the step of producing stimulation signals can produce stimulation signals which stimulate selected electrodes based on their position to cause stimulation of specific nerves within the spinal cord.

The method may further include the step of percutaneously explanting the electrodes after the treatment. An advantage of small devices with flexible electrode arrays is that they can be explanted without significant difficulty and without causing injury to the patient at or around the implantation site. Therefore the electrodes can be explanted, for example if an alternative therapy is being used, or if the electrodes need to be replaced or repositioned.

Unless indicated otherwise, any of the features (including the optional or preferred features) described in relation to one of the above aspects are equally applicable in combination with the devices, systems and methods of any of the other above-described aspects.

The invention is described below, by way of example, with reference to the accompanying figures in which:

Figure 1 shows the design and characteristics of implantable devices used in embodiments of the present invention and the surgical process of implantation forming part of embodiments of the present invention;

Figure 2 shows a system according to an embodiment of the present invention with devices implanted to sites on the spinal cord of a patient;

Figure 3 shows the steps in the manufacture of the devices of Figure 1;

Figure 4 shows the use of the devices of Figure 1 as a neurostimulator;

Figure 5 shows the principles of and testing of circumferential spinal cord recording using the devices of Figure 1;

Figure 6 shows various steps and results from the analysis of recorded spinal signals;

Figure 7 shows the principles of bioelectronic bypass of an acute spinal cord injury site;

Figure 8 shows the design and characterisation of alternative implantable devices used in embodiments of the present invention;

Figure 9 shows results from foreign body reaction analysis associated with implantation of the devices of Figure 8;

Figure 10 shows recording of electrode signals from target nerves using the devices of Figure 8;

Figure 11 shows the intended implantation of devices of Figure 8 and the recorded signals from the nerves using those devices;

Figure 12 shows the results of stimulation testing using the devices of Figure 8; and

Figure 13 shows the functional elements of a system according to an embodiment of the present invention.

It has been demonstrated that direct stimulation of anterior motor neurons via ventral stimulation is more efficient in muscle activation and allows a greater degree of selectivity [6]. This finding has led to recent efforts to develop ventral SCS devices [7, 8]. Furthermore the spinal cord proximal to a site of injury, rather than external algorithms or brain cortex, represents a more viable and patient friendly target for neural recording and decoding of motor intent [9, 10]. However, challenges exist for both the anatomical approach to the ventral spinal cord and the use of materials that limit iatrogenic neural injury. Furthermore, to date spinal cord signals have yet been used as control signals for closed loop SCS, due to limitations in recording and stimulation techniques, which only allow coverage of limited areas of the spinal cord.

By harnessing advances in thin-film bioelectronic devices that more closely match the mechanical stiffness of neural tissue [11, 12], embodiments of the present invention include non-penetrating conformal bioelectronic arrays capable of interfacing with the spinal cord circumferentially. This has been shown to be possible without causing iatrogenic spinal cord injury in rats. As set out further below, this approach allows comprehensive recording of neural signals around the spinal cord with high spatial and temporal dimensionality, which provides for the possibility of whole cord signal analysis and simultaneous descending and ascending tract measurements. Embodiments of the invention combine recording and stimulation and use these circumferential spinal cord implants to enable direct, low-latency spinal cord electronic bypass to restore hindlimb motion in acute SCI. This has been tested in rat models and the translation to human cadaveric spine models has also been tested.

*Device and Surgical Design*

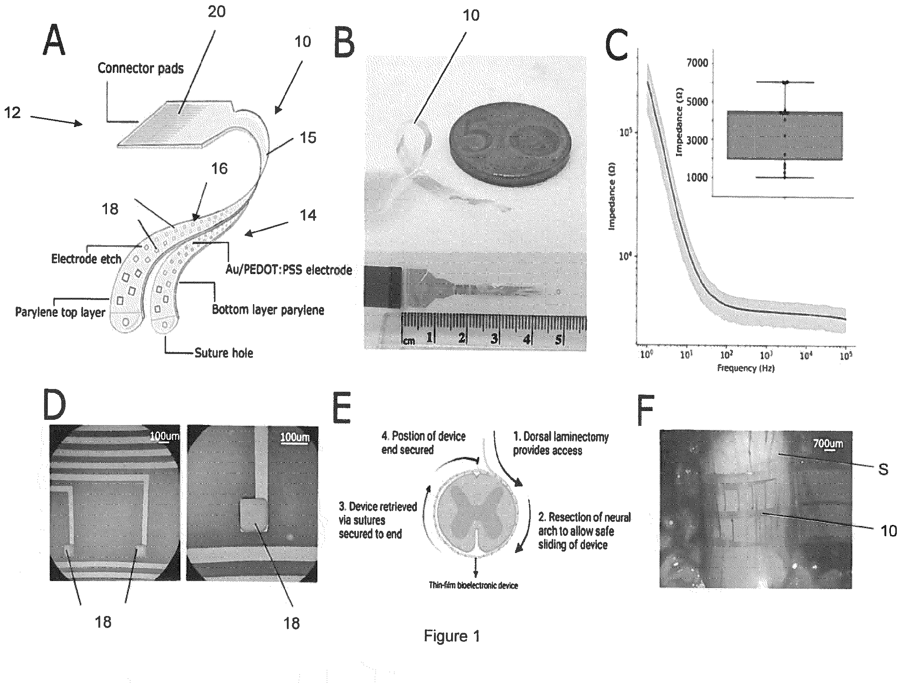

Figure 1 shows details of a device used in embodiments of the present invention, along with further aspects associated with the device and the surgical design.

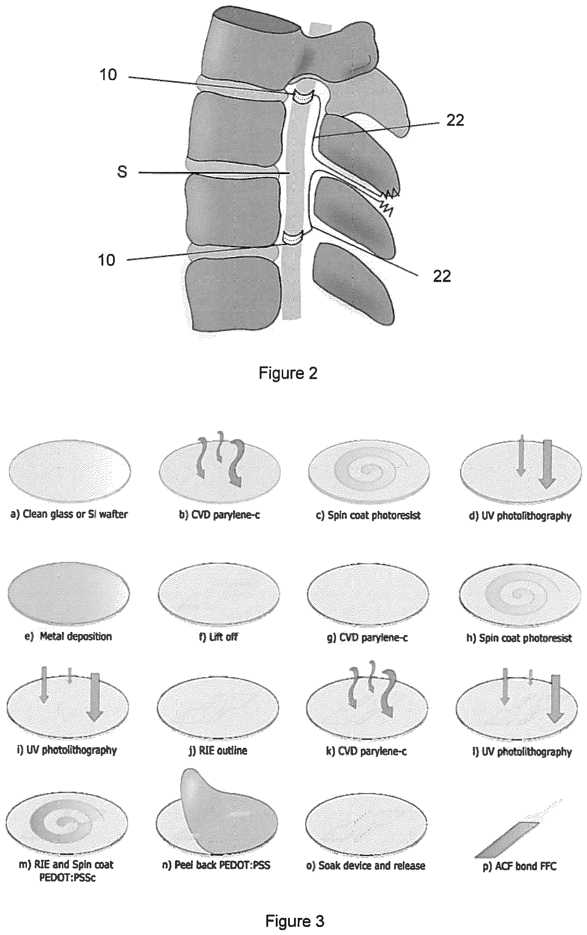

Figure 1A is an exploded illustration of a microfabricated device 10 as used in embodiments of the present invention. The full fabrication of this device is described further below in relation to Figure 3. The device 10 has a proximal end 12 and a distal end 14. Figure 1B shows the device illustrated in Figure 1A with scaling references, and also demonstrating the looped flexibility of the device.

To enable a circumferential implantation around the spinal cord, the distal end 14 of the device 10 has a thin, flexible electrode array 16 consisting of thirty-two electrodes 18 arranged in a staggered, linear configuration. Such a configuration maximizes the capture of neural signals distributed around the spinal cord whilst minimizing cross-talk contamination [13]. The device is appended by a reinforced gold ring that allows a size 7-0 polypropylene suture (Ethicon Inc., Raritan, NJ, USA) to pass the device through the ventral aspect of the spinal cord. The array 16 has a parylene-C substrate with titanium and gold electronics and is fabricated using standard photolithography techniques [14].

The flexibility of the distal end 14 of the device 10 is preferably such that it can conform to and/or wrap around the portion of the patient that it is intended to interface with. For devices which are intended for interfacing with the human spinal cord, this means that a bend radius of no more than 0.5 cm is preferable (being the radius of smaller human spinal cords), and preferably the bend radius is no more than 0.25 cm. As the devices 10 manufactured and tested for the present purposes were designed for interface with the spinal cords of rats, they were comfortably manufactured with much smaller bend radii. For devices which are intended for interfacing with individual nerves, the bend radius is preferably in the micron range, for example no more than 500 $\mu$m, preferably no more than 100 $\mu$m and ideally smaller.

Bend radius, which is measured to the inside curvature, is the minimum radius that a component (in this case the device) can be bent in at least one direction without damaging it. The bend radius as defined here refers to elastic deformation as opposed to plastic deformation such that a device bent under an applied force to a radius greater than the minimum bend radius would return at least part way to its original shape with the removal of the applied force. In other words, in these embodiments, the device of this aspect can be bent to an inside curvature of 0.25cm (spinal cord interfacing devices) and still function exactly as it did prior to bending.

Conducting polymer Poly (3,4-ethylene) dioxythiophene (PEDOT) with poly(styrene sulfonate) (PSS) acting as counterion to PEDOT was used to decrease the impedance of the electrodes and thus enhance performance in both recording and stimulation [15]. Figure 1D shows an optical image of the device electrodes 18, showing the PEDOT:PSS coating. The footprint of the electrode interconnect 15 is minimized to allow the device to be implanted through the interval between the exiting nerve roots. The circumferential spinal cord interface devices 10 were manufactured in a range of dimensions (8.90mm, 9.90mm and 10.90mm lengths) to allow adaptation to variations on spinal cord anatomy in order to implant the devices that maximized electrode coverage of the spinal cord circumference. It will be noted that these specific dimensions were chosen for the studies in rats, as discussed further below. The dimensions of devices for human use are chosen accordingly. In particular, for devices which are intended to wrap circumferentially around human spinal cords, the distal end of the device 14 (having the electrode array 16) is preferably at least 3cm in length, and may be between 3cm and 5cm in length (to circumferentially wrap around a human spinal cord which typically has a diameter of 1 to 1.5 cm). For devices which are intended to wrap around nerves, the devices will be corresponding smaller in size. The number and configuration of electrodes 18 in the electrode array 16 is also chosen accordingly. The electrode size was 0.1 x 0.1 mm in a square configuration.

The devices were characterized in vitro using electrical impedance spectroscopy (EIS). Figure 1C shows the EIS measurements from the device 10 with n=13 electrodes from 2 devices. The inset in Figure 1C shows the range of impedance values seen at 1kHz with individual device electrodes demonstrating low impedance (1-6kΩ) at 1kHz.

The devices were implanted proximal to the rat L1 anatomical level to interface directly with the spinal cord instead of the exiting nerve roots which are predominantly distal to the L3-L4 level. Figure 1E shows a process flow of the surgical steps and device placement steps which were performed before the recording or stimulation experiments discussed below were carried out. Figure 1F is an interoperative image of the device 10 wrapped around the longitudinal axis of the spinal cord S. The bottom of the image shows the L1 superior facet.

To detect any potential iatrogenic spinal cord injury during implantation of the devices, neuromonitoring was performed at regular intervals during the implantation which did not show increases in threshold amplitude required to evoke motor evoked potentials (MEP) [16]. Following the resection of the vertebral laminae the device was inserted using a leading 7-0 polypropylene suture (Ethicon Inc., Raritan, NJ, USA). After successful device implantation, the electrode located in the midline of the spine was used to calibrate the topographical interpretation of the acquired signals.

Figure 2 shows, schematically, a system with two devices 10, such as those previously described, located in positions wrapped around the spinal cord S of a patient. These locations can be chosen to be either side of a lesion which has caused SCI in the patient. The devices are connected by wires 22 to a controller (not shown).

*Manufacturing the Devices*

Figure 3 shows the fabrication steps in an example method of making a device 10 such as that described above in relation to Figure 1. The steps of the method are described in more detail below.

The first parylene deposition is performed using the PDS 2010 Labcoter™ 2 (Specialty Coating Systems Ltd, Woking, UK) on a 4-inch silicon wafer (Microchemicals GmbH, Ulm, German), with 4g of parylene-C dimer to form layers of 2 μm. This is followed by the first photolithography for the gold electrode patterning using the MA/BA6 mask aligner (SUSS MicroTec, Garching, Germany), spin-coating the AZ® nLOF 2035 negative photoresist MicroChemicals GmbH, Ulm, Germany) at 500 revolutions per minute (rpm), 1000 rpm for 5s and at 6000 rpm, 8000 rpm for 30s to achieve a thickness of 2.00-2.50 μm, followed by soft baking at 110°C for 60s, ultraviolet light exposure for 7s and post-baking at 108°C for 150s. AZ® 726 MIF MicroChemicals GmbH, Ulm, Germany) is used as a developer for between 20-30s before distilled water wash and drying with compressed nitrogen.

The samples are activated using oxygen plasma under vacuum (60s at 100 W and 0.6 mbar O2) using the Plasma Pro 80 RIE (Oxford Instruments Plasma Technology, Bristol, UK) to improve adhesion between parylene and the electronic components, titanium (Ti) and gold (Au) which were deposited via electron-beam evaporation (Kurt J. Lesker Company, Jefferson Hills, PA, USA). 10-nm Ti was evaporated under vacuum (<6 x 10-6 torr) and allowed to rest for 10 min, followed by 100-nm Au deposition. The sample is then left in acetone for 30-60 min and subsequently lifted off carefully with a swab stick. Finally, they are rinsed with isopropyl alcohol (IPA) and DI.

The second layer of parylene is deposited using the same steps described above. This is followed by photolithography for the outlines of the devices using the MA/BA6 mask aligner (SÜSS MicroTec, Garching, Germany), spin-coating the AZ® 10XT (MicroChemicals GmbH, Ulm, Germany) negative photoresist at 500rpm, 1000rpm for 5s and at 3000rpm for 30s to achieve a thickness of ~6.00μm, followed by soft baking at 110°C for 120s, ultraviolet light exposure at 6 steps of 5s with a 5s interval between the steps. The sample is subsequently developed in AZ® 726 MIF (Micro-Chemicals GmbH, Ulm, Germany) developer for 6-7min and washed with DI. Reactive-ion etching (RIE) is then completed with the Plasma Pro 80 RIE (Oxford Instruments Plasma Technology, Bristol, UK) at an etching rate of 200nm/min. This is followed by the third layer of 2μm sacrificial parylene deposition separated by soap (2% Micro-90 Cleaning Solution, Cole-Parmer Instrument Company Ltd, St. Neots, UK), followed by further photolithography to expose the electrodes and contact pads using AZ® 10XT (MicroChemicals GmbH, Ulm, Germany), spin-coated for 30s at 3000rpm followed by baking for 120s at 110°C. The samples are then exposed and developed, followed by RIE as described above, until metal exposure was completed.

The conducting polymer Poly (3,4-ethylene) dioxythiophene (PEDOT) is combined with Poly(styrene sulfonate) (PSS) which acts as counterion to PEDOT to increase the conductivity of the electrodes [15]. The PEDOT:PSS is prepared according to previously described methods34, with 19ml of CLEVIOS™ PH 1000 PEDOT:PSS (Heraeus Holding, GmbH, Hanau, Germany) mixed with 1ml of Ethylene Glycol (EG) which improves film conductivity and 2 drops of 4-Dodecylbenzenesulfonic acid (DBSA) to increase the homogeneity of the spin-coated film. The mixture is ultra-sonicated for 10-15min and filtered through 1.2μm Polytetrafluoroethylene (PTFE) filters.

This is followed by spin-coating of the PEDOT:PSS layer at 3000rpm for 30s, baking for 60s at 110°C and two cycles of spin-coating at 1500rpm for 30s and baking at 110°C for 60s. Lastly, the sacrificial parylene layer is peeled off to reveal only the PEDOT:PSS layer and the samples are hard baked for 1 hour at 130°C. The devices were packaged using 33-channel flat flexible cables (Mouser, UK) with a bonder (Fineplacer, Finetech GmbH & Co. KG, Berlin, Germany) using a 5 $\mu$m anisotropic conductive film (ACF, Jetro, Japan).

*Using the Device to Stimulate the Spinal Cord Circumferentially*

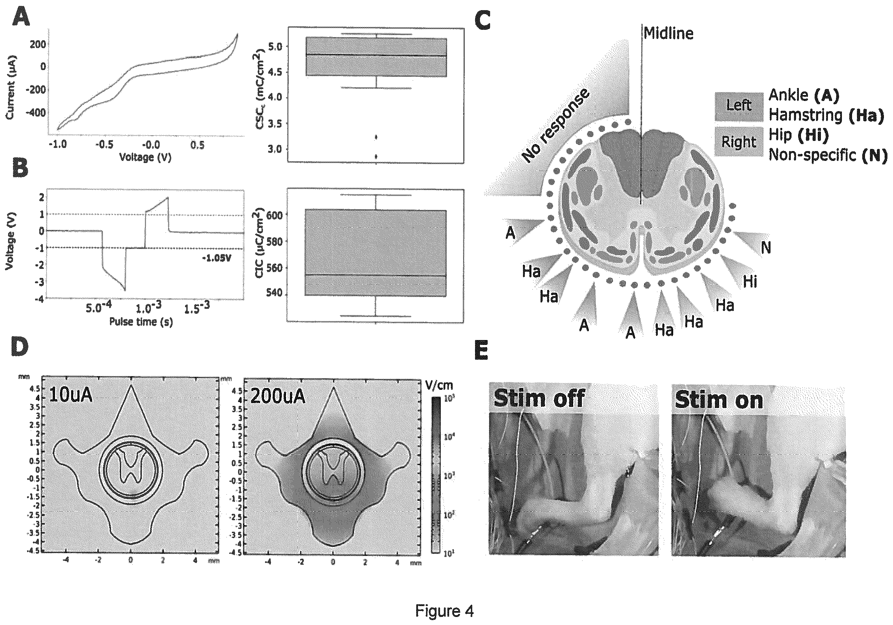

A first set of tests were performed with a device such as that described above being used to selectively stimulate the spinal cord. These tests investigated the application of spinal cord stimulation around the perimeter of the spinal cord but were especially concerned with the effects of tonic stimulation on the ventral side of the spinal cord. Firstly devices were characterized for charge storage and charge injection capacity. Using microelectrodes, it is important to ensure that the charge delivered is not damaging to the neural tissue. The application of PEDOT:PSS on a conductive metal (Au) electrode dramatically increases both charge storage and charge injection capacity [17] and allows for wider stimulation limits. These data are summarized in Figures 4A and 4B respectively.

Figure 4A shows a representative cyclic voltammogram (CV) trace and derived charge storage capacity (CSC) data (n=10). Figure 4B is a representative transient voltage graph following electrical pulse and derived charge injection capacity (CIC) data (n=8).

Further, the application of the device to recruit specific motor movements, ideally located in the ankle, hamstring and calf of the animal under investigation, was investigated. Other investigation covered whether stimulation at the ventrolateral threshold amplitude level would elicit a motor response when stimulating the dorsal area of the spinal cord which it did not as shown in Figure 4C.

Figure 4C shows the results of a stimulation study in a rodent model showing specific control over several lower limb motor groups and a lack of action in the dorsal sections of the spinal cord. Figure 4E are two still extracts from a live video recording of lower limb muscle flexion during stimulation of the spinal cord at 20 $\mu$A

The stimulation experiments (Figures 4C and 4E) were supplemented with in silico data (Figure 4D) looking at current spread to infer likely recruitment at various points across the spinal cord. Figure 4D shows the results of finite element simulation of the electric field magnitude in the rat spine. The simulations were carried out across multiple peak current levels, here minimum and maximum currents used during the in vivo experiments, 10 and 200 $\mu$A respectively.

Using tonic stimulation patterns of 50-100 Hz with 10-20 pulse trains, smooth movements were elicited in a controlled bilateral manner. Ankle, hamstring, calf, hip and flank muscles were recruited as shown in Figure 4C.

To perform the simulation modelling, the electric field distribution in the rat spine was analysed by conducting a three-dimensional finite element simulation using COMSOL Multiphysics 6.0 (electric current interface of AC/DC module) solving the point form of Ohm's law assuming current conservation. Fixed DC electrode injection currents of 10 $\mu$A or 200 $\mu$A at electrode surface were simulated. The model geometry is a three-dimensional extension of the two-dimensional geometry which is based on MRI scans of a rat T10 vertebrae 41. The geometry consists of muscle, bone, epidural space, dura, cerebrospinal fluid, white and grey matter domains. The device is represented by a 4 $\mu$m thick ring of a low conductivity (0.001 S/m) material representing parylene-C with (100 $\times$ 100) $\mu$m$^2$ sized electrodes placed directly on the dura surface. All domains were approximated to be perfectly isotropic conductors. The spine model was embedded in a (50 $\times$ 50) mm$^2$ cube with the electrical conductivity of muscle tissue and with grounded outer boundaries. The figures show the electric field magnitude in the plane normal to the electrodes.

*Using the Device to Record Spinal Signals Circumferentially*

The same device as previously described and as used for the stimulation investigations was then used to record neural signals around the spinal cord.

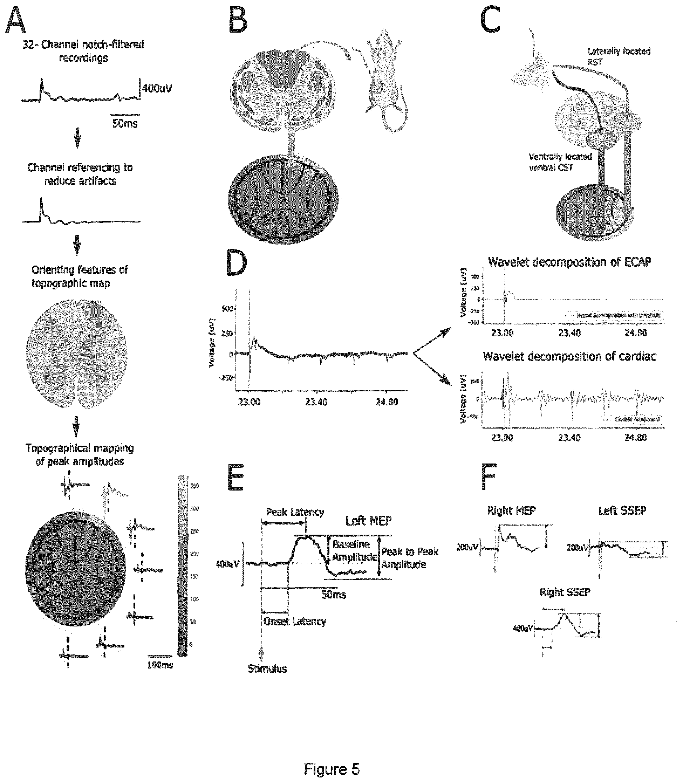

Following safe implantation of the devices as evidenced by preserved hindlimb MEP responses, acute electrophysiology was performed by triggering the sequential activation of bilateral MEP via the hindlimb motor cortex and somatosensory evoked potentials (SSEP) via the sciatic, tibial and peroneal nerves which were recorded by the device. The circumferential placement of the electrodes around the spinal cord enables a comprehensive topographic representation of the acquired signal amplitudes at each sampled interval, as shown in Figure 5A.

Figure 5A shows a process flow of topological map creation: First, recording of raw signals from 32 channels around the spinal cord is carried out. Second, channels are referenced and filtered to remove artifacts. Third, the features are orientated on a map based on electrode position and coverage. Fourth, a full topological map is created based on the peak amplitude of the neural signals.

This spinal cord topography capitalizes on the ordered arrangement of ascending and descending tracts traversing the spinal cord. This is illustrated when stimulation of the left sciatic nerve produces a hotspot on the spinal cord topographic map corresponding to the expected location of the dorsal column as shown in Figure 5B, which is a topological map created from somatosensory evoked potential recordings.

The density of electrode recordings around the spinal cord also allows better differentiation of various hotspots around the spinal cord. The descending motor pathway is mediated by the rubrospinal and corticospinal tract in rats [18-20]. This phenomenon is illustrated by spinal cord topography demonstrating two locations of peak activation simultaneously following MEP stimulation, corresponding to the approximate location of these descending motor axons. Figure 5C shows how motor evoked potential (MEP) recordings demonstrated peak amplitude hotspots predominantly on the ventrolateral and lateral areas of the spinal cord corresponding to approximate locations of the ventral corticospinal tract (CST) and rubrospinal tract (RST) respectively.

Besides using channel referencing to isolate neural signals, it was also possible to automatically isolate neural signals from instrumental and biological noise generated by echocardiogram (ECG) signals and respiratory activity using wavelet decomposition as a de-noising technique [21]. This is illustrated in Figure 5D, which shows the extraction of the cardiac and neural components of the raw signal. Using either method of neural signal extraction allows signal waveforms to be studied to obtain conventional measurements of latency, amplitude, and neural conduction velocity in a low noise environment. Figure 5E shows how recorded compound action potentials (CAPs) can be analyzed in greater detail, here details of waveform analysis are provided. Figure 5F shows both left and right SSEP and MEP waveforms from representative electrodes on the spinal cord are shown.

A subset of the raw recordings was independently analysed using wavelet decomposition to validate the ability of this method to automatically isolate the neural signals from biological and instrumental noise, reducing the manual workload that accompanies the previously described processing. A direct approach to identify the ECAPs was taken by performing a denoising multiresolution analysis of the signal using discrete wavelet transformation (DWT) as originally proposed by Diedrich et al. [21], as this choice outperforms other alternatives when working with similar neural signals.

Orthogonal Daubechies of order 5 using 3 levels of decomposition were empirically chosen based on their similarity to the ECAPs of interest and to filter out low frequencies. The hard-thresholding regular wavelet de-noising technique proposed by Donoho [45]] was chosen, which essentially applies a threshold to the coefficients at each decomposition level in the orthogonal time-frequency domain to further isolate the structures of interest, before transforming them back into the original domain denoised. The threshold was calculated as:

$$Th = \sigma \sqrt{2 \ln(N)} \qquad (1)$$

where $\sigma$ is the standard deviation of the Gaussian noise and N is the number of samples in the coefficients. Because orthogonal wavelets were used, the length of the coefficients at all decomposition levels is the same as in the original signal. At each level '$l$', the $\sigma$ was calculated as:

$$\sigma_l = \frac{median(|c_l|)}{0.6745} \qquad (2)$$

Where $c_l$ was the value of the wavelet detail coefficients at each level, and 0.6745 is the 75th percentile of the standard normal distribution [45]. Then detailed coefficients were hard-thresholded for reconstruction of the de-noised signal as:

$$y = \begin{cases} x \; if \; |x| > Th \\ 0 \; if \; |x| < Th \end{cases} \qquad (3)$$

Spikes on the reconstructed signals were then identified by using a detection threshold as defined in Eq. (1). A spike window centred around the detected peaks of 4ms length was extracted to obtain the corresponding waveforms. The cardiac component of the signal was also isolated by applying continuous wavelet decomposition using the Ricker wavelet family at a 5-ms scale.

*Analysis of Circumferential Spinal Cord Recordings*

Integrating the abundant neural signal data in both the time and spatial domains allows for the identification of unique topographic signatures that reflect the dominant spinal cord tract activated. Sequences were produced of alternating left and right, MEP and SSEP stimulation, producing t patterns corresponding to the expected location of spinal cord tract activation. This allows a clear visualization of where the tracts are activated and produces unique topographic signatures that allow for accurate classification of the source signals.

To perform this testing, the recording device was connected to a 32 channel recording head stage (Intan Technologies, Los Angeles, CA, USA) via a custom built omnetics/ZIF connector PCB and flexible flat cable, signals were acquired at 30kHz through the RHS 2000 stimulation/recording controller (Intan Technologies, Los Angeles, CA, USA) with a 50Hz notch filter to reduce line noise from electronic current sources. The device was grounded via a stainless steel wire implanted into the paravertebral muscles and impedance measurements were obtained.

MEP stimulation was performed using a 50μm tungsten microwire electrode insulated with polyamide (California Fine Wire Company, CA, USA) secured to a stereotaxic frame and grounded to a stainless steel cerebellar screw. The microwire was guided to the cortical areas representing hindlimb motor function as determined by previous mapping studies [42]. Motor cortex stimulation was performed with a 200Hz train of 20 alternating square wave pulses of 100ms pulse width to maintain a short stimulation duration [43], starting from 50μA and increasing in 25-50μA steps until the minimum stimulus energy needed to elicit a barely perceptible muscle contraction, referred to as the threshold level, was determined. If a satisfactory MEP was not obtained, the area of motor cortex stimulation was shifted 3-5mm in depth and/or rostro-caudal direction.

SSEP stimulation was performed using a customized nerve hook electrode applied over the sciatic, peroneal and tibial nerve, anchored via silicon sealant (Kwik-Sil™, World Precision Instruments, Hitchin, UK), and grounded to a stainless steel wire placed at the base of the tail. The stimulation protocol consisted of a single alternating square wave pulse of 100ms, starting at 50μA and increased in 10-25μA steps until the threshold level was determined.

EMG was recorded using electrodes made out of stainless steel 25 gauge needles, inserted into the quadriceps muscles and grounded to a stainless steel wire placed at the base of the tail. Trains of alternating left/right, SSEP/MEP stimulation was applied to provide data for analysis and machine learning, lasting approximately 50-70s per session with 1s between each stimulation pulse. All stimulation protocols were applied using the RHS 2000 stimulation/recording controller (Intan Technologies, CA, USA).

The raw signals were imported into MATLAB (R2021a) without further filtering to preserve key time domain information. Active channels were referenced to dormant counterparts to reduce electrocardiogram, respiration, stimulation, and movement artifacts. Evoked compound action potentials (ECAPs) were identified, defined as time-locked positive waveforms following a stimulus [44], using an automated peak finder and verified by visual inspection of the waveforms to ensure that erroneous signals were not included in analysis. As the signals in the 32 channels were of varying amplitudes at each sampled interval, the amplitudes in each channel around the spinal cord could be plotted in a topographic heatmap using a custom MATLAB script, visualizing the areas of activation at each sampling interval. This topographic heatmap generated a spatial signature that approximately corresponded to the area of underlying spinal cord tract activation and could be used to correlate with the source of evoked potential.

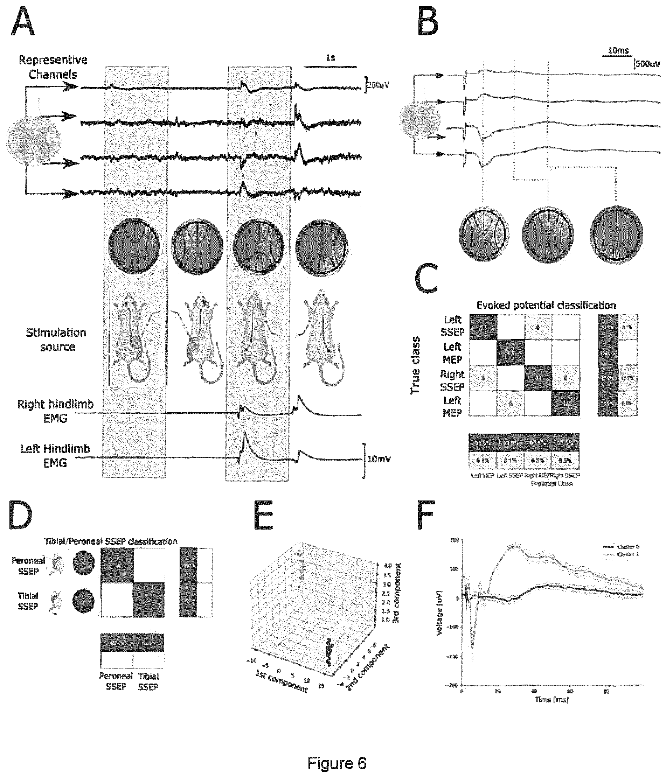

Figure 6A shows representative recordings across four quadrants of the spinal interface covering left dorsal, left ventral, right dorsal and right ventral areas of the spinal cord during SSEP and MEP stimulation events. Topographic heatmaps at a single point in time as well as associated EMG recordings are also shown.

The high sampling rate of recordings obtained also enables the interrogation of peri-event potential amplitudes following an evoked potential, highlighting areas of shifting peak amplitudes around the spinal cord. After evaluation of the topographical data following SSEP stimulation of the sciatic nerve, it was possible to identify multiple signal peaks consistent with previous studies [22, 23]. These peaks, however, were of different relative amplitudes in the various channels, which demonstrates that the topographic hotspot shifts with time after stimulation.

Figure 6B is a diagram from the 4 representative channels demonstrating how recorded signals evolve through time following stimulation of the sciatic nerve. This phenomenon was reflected in the majority of the recordings, and the inventors believe that this shift reflects complex interneuron interactions with dorsal column neurons activated and subsequently sending impulses to ventrally-based corticospinal and spinothalamic neurons.

The circumferential spinal cord recordings generate topographic peak signal amplitudes distributed over the 32 channel recordings at each sampling interval unique to the various sources of evoked potentials. Using a thresholding method based on the peak amplitude means allows feature extraction of the signals in preparation for data analysis. This allows for the use of a supervised machine learning approach to identify these characteristics to classify the source of evoked potentials using a KNN algorithm. This resulted in a classification accuracy of 93.8% when categorizing source signals according to the following categories: left MEP, right MEP, left SSEP and right MEP. Figure 6C is a matrix of classification showing positive and negative hit rate of left/right SSEP/MEP stimulation events.

The density of signal information obtained from the circumferentially placed electrode also enabled further differentiation of the source signal to the level of peripheral nerve branches. The tibial nerve, supplying and receiving sensorimotor input from the anterior compartment of the leg, was stimulated in an alternating sequence with the peroneal nerve reflecting the posterolateral compartment of the leg. The stimulation of these two sciatic nerve branches generated unique spatial patterns represented by the 32 channels on the electrode, allowing classification of the source signals. Figure 6D is a classification matrix showing tibial vs peroneal stimulation events.

An automated strategy to extract the signal features for classification was also investigated, using linear and non-linear dimensionality reduction methods. Following wavelet decomposition to identify cardiac and neural peaks, this allowed an unsupervised machine learning classification algorithm to identify distinct clusters allowing comparable classification accuracy of tibial/peroneal nerve stimulation source signals, albeit at a higher computational cost and processing time. This provides a secondary method of classifying peroneal and tibial alternating stimulation as shown in Figure 6E which is a 3D graphical representation of the principal component analysis showing how tibial and peroneal stimulation can be clustered based on waveform shape for a single dorsally positioned electrode. A representative waveform comparison from a dorsally located electrode is shown in Figure 6F, which shows the temporal evolution of the averaged clustered waveforms for a tibial vs peroneal stimulation event from a single dorsally positioned electrode.

The high-dimensional raw signal data obtained from the 32 channels required further processing before classification modelling. Two strategies were implemented. To begin with, feature extraction was performed, characterizing the peak amplitude [46] of each ECAP. A threshold was then determined based on the mean value of the recorded peak amplitudes for each of the 32 recording channels, effectively converting continuous raw signal data into categorical variables.

In the second strategy, the high-dimensional space corresponding to the waveforms extracted after wavelet denoising was reduced using linear and nonlinear dimensionality reduction methods. In particular, principal components analysis [47, 48] (PCA) and uniform manifold approximation and projection [49] (UMAP) were applied and compared. UMAP is a non-linear method that showed to outperform the linear PCA in datasets were non-linear dependencies were remarkable, although at a higher computational cost.

Dimensionality reduction was necessary as modelling with high-dimensional raw signal data led to overfitting and reduced predictive performance [50], which can be solved with feature extraction and dimensionality reduction.

The processed data was used to train the machine learning model, partitioning 60% of the data into a training set and 40% of the data into a classification set. For supervised machine learning, the k-nearest neighbors (KNN) algorithm [51] with automatically optimized hyperparameters (k=1, city block distance metric) was used in MATLAB (2021a). The classified data was aggregated to generate a confusion matrix to calculate overall classification accuracy and classification loss rates for each source of evoked potential. Tibial versus peroneal SSEP classification was aggregated separately as they were subsets of left/right SSEP. Finally, an unsupervised method based on KNN with k=2 (for tibial versus peroneal SSEP dataset) or k=4 (for alternating left/right, SSEP/MEP stimulation dataset) was used to automatically identify distinct clusters.

*Establishing a Spinal Cord Bypass Following Complete Transection*

The dual functionality of the recording and stimulating capabilities of the devices described and illustrated above is applied in embodiments of the invention by using neural signals recorded by one device positioned around the spinal cord above the site (i.e. closer to the brain) of an SCI as triggers to produce targeted spinal cord stimulation in a second device below the site (i.e. further from the brain), in order to functionally bypass the site of acute spinal cord injury.

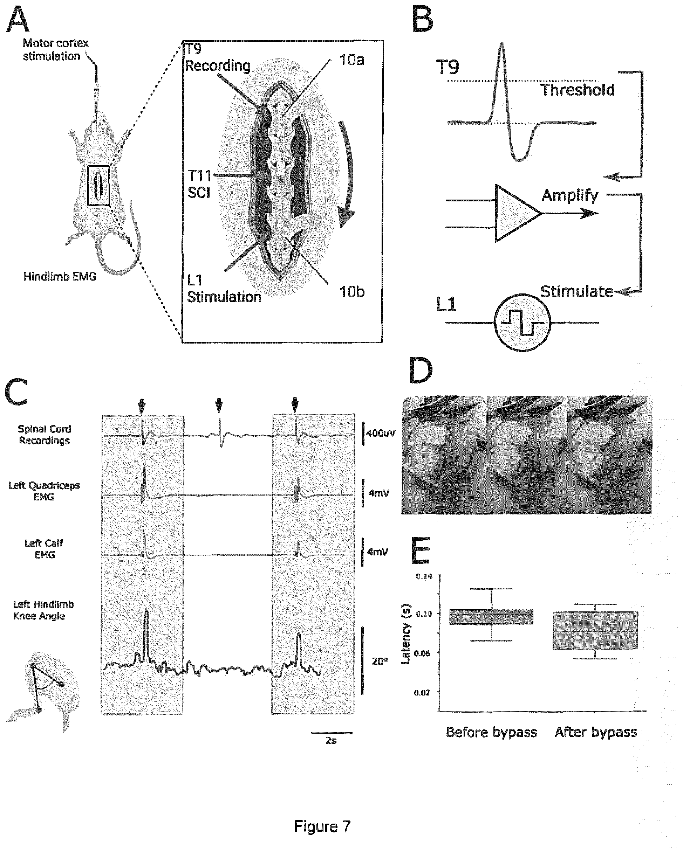

This approach required the implantation of two devices 10a, 10b at the T9-10 and T12-L1 levels respectively as shown in Figure 7A, using neuromonitoring to ensure that the implantation did not lead to an iatrogenic SCI. Intermuscular EMG probes were connected to the lower limbs. MEPs are generated using a microwire placed in the motor cortex. An acute transection of the spinal cord was performed in between the two devices 10a, 10b with the lack of MEP recorded in peripheral electromyography (EMG) traces demonstrating the discontinuation of descending motor signals.

To produce a low-latency communication between the recording and stimulating device, an ECAP threshold detection method was used which was not computationally intensive. This allowed the proximal, recording device 10a to detect the peak of the MEP action potential and trigger the distal, stimulating 10b device to effect hindlimb movement. The workflow is shown schematically in Figure 7B: a signal is recorded at T9, amplified, an amplitude threshold is applied and used to stimulate a corresponding electrode below the injury

The stimulation algorithm can be modulated to produce hindlimb movement with varying muscle activation patterns. The electronic bypass can be deactivated and reactivated readily to allow differentiation between states of rest and activity. For this technique to function as a physiological bypass, the latency between the recording and stimulation algorithm should aim to be comparable to the latency of neural transmission along the spinal cord.

Figure 7C is example data showing recorded signals from the ventral part of the spinal cord and quadricep/calf EMG waveforms following sub injury stimulation. Hindlimb angles were calculated from marker less kinematic data which correspond to EMG and MEP waveforms.

Figure 7D is a time series video overlay of lower limb activation using the spinal bypass approach described above.

Figure 7E shows the MEP latency calculated before and after the spinal cord injury was established (n=6).

Following implantation of both recording and stimulating devices 10a, 10b, control signals for bypass in acute spinal cord injury were generated using Intan RHX data acquisition software (Intan Technologies, Los Angeles, California). By examining action potentials generated around the spinal cord with motor cortex stimulation and confirming their approximate positions on the spinal cord, the selected channels were referenced to reduce ECG and respiratory artifacts to de-noise the action potential waveforms. These waveforms can be detected via their rising edges and their amplitudes amplified using the native Intan RHX software to generate trigger signals for spinal cord stimulation to reproduce the disrupted hindlimb movement following the acute SCI.

*Human Translation*

For human translation, the devices were scaled to human dimensions to explore surgical implantation feasibility. A parylene-C based dummy device was used, that is, a device with the same dimension and mechanical characteristics as a human-scaled version of the devices described above, but with none of the electrical connections required to function as an electrical interface. Firstly, the spinal cord was reconstituted using a saline pump as previously reported [12]. A laminectomy was performed at the thoracic T9 to T10 level. Following this a midline durotomy was performed to expose the parenchyma. The dura was gently retracted using sutures to allow the surgeon to operate in the space. The parylene device was then passed laterally down the side of the spinal cord before being withdrawn from the opposite lateral side, following passage of the device under the ventral surface. The end result is the device being wrapped around the spinal cord completing the surgery. When using an electrically active device at this point the interface could be used to record signals from or stimulate the spinal cord.

The devices were also tested during a human cadaver session at the Evelyn Surgical Training Centre, Cambridge (UK). Fresh-frozen specimens were used. To achieve the spinal reconstitution with saline, the dura was first exposed by incision above the cervical part of the spinal cord, removal of spinous process and full laminectomy at the C4-6 vertebrae. An incision was made in the dura with a gauge-18 needle, a polyethlye tubing (OD=1.4mm) was inserted into the incision and secured using a purse string suture (Prolene 4-0), a small amount of cyanoacrylate glue (Loctite) was dispensed around the tube to ensure an adequate seal. The tubing was connected to a flow pump (Flowsteady, model 200). The pump was set to 140 mmHg at a rate of 1.5 L/min. After 30 seconds the dura could be seen to expand, after 60 seconds liquid could be seen emerging from the epidural space, indicating either a small leak of the dura or fluid displacement as the dura expanded. The pump was reduced to a pressure of 70 mmHg and 0.5 L/min. The seal was monitored for at least 30 minutes before the first implantation to ensure that the seal held, and the dura did not leak or expand further.

A midline incision was performed over the thoracic T9 to T10 vertebra with the dissection taken down to bone. A wide laminectomy was then performed from pedicle to pedicle, followed by a midline durotomy. Sutures were used to retract the dura gently and allow access to the dorso-lateral spinal cord. The dentate ligaments were released to allow the device to slide unopposed around the spinal cord. To introduce the device without impinging on the spinal cord, micro-forceps were used to guide the device laterally along the dura until it reached the ventral aspect of the spinal cord. The device was then advanced until the tip could be seen and retrieved at the contralateral side of the spinal cord.

*Discussion and developments*

The investigations described above have demonstrated that interfacing circumferentially around the spinal cord enables comprehensive representation of neural signals, generating control signals to trigger targeted spinal cord stimulation and ultimately facilitates electronically bypassing an acute SCI. The devices used are formed from thin, flexible bioelectronics that can be implanted around the spinal cord without causing neural damage. The techniques are made possible using non-penetrating arrays which limit injury when compared to stiff and bulky current SCSs and minimize foreign body reaction secondary to chronic implantation [24].

Epidural electrodes minimize iatrogenic spinal cord injury [28] whilst recording information in the ascending and descending tracts of the spinal cord, located peripherally in the white matter. Previously, epidural recording of the ventral and ventrolateral tracts was challenging due to the anatomical obstacles (vertebral body, neural arch) preventing safe insertion of recording probes without causing spinal cord injury, restricting the investigation of spinothalamic and ventral corticospinal tracts, the latter of which is the dominant motor pathway in humans. The surgical techniques described above overcome these anatomical obstacles by judicious resection of the neural arch to slide the devices safely under the ventral spinal cord. Though a necessary part of the implantation process for a rodent scale device, this may not be required for human placement due to the greater size.

Owing to the somatotopic arrangement of hindlimb muscle groups around the spinal cord, it was possible to achieve selective activation of specific hindlimb muscle groups. This was achieved by directly stimulating the ventral and lateral epidural spinal cord where the ventral corticospinal tract and anterior motoneurons are located. Additionally, the pattern of muscle activation can be modulated by varying the stimulation parameters to produce gentle, sustained or explosive movements [29]. This can empower neuroprosthetics to enable movements beyond the current walking or stepping targeted SCS algorithms, allowing SCI patients to perform specialized activities.

In the above-described methods, a latency of the communication between the recording, decoding and stimulating devices has been achieved which is comparable to physiological neural conduction latency. This enables more natural movement patterns for the user [30]. In further embodiments, decoding motor intention may require more complex filtering and classification algorithms, and potentially learned models which are trained to be unique to an individual patient [10].

Whilst acute neuromonitoring confirmed that acute implantation of these devices did not lead to SCI, the dynamic interactions between the device and the spinal cord may lead to injury. To ensure long-term safety of these circumferentially implanted devices such interactions can be mitigated by the choice of thin, flexible materials that can accommodate to the movement within the spinal cord [31].

The above studies have been conducted in rates. However, the somatotopic arrangement of the spinal cord tracts in rats differ from humans [32]. The skilled person will thus appreciate that the precise configuration of the devices need not follow the examples above and that alternative arrangements may be preferable for engagement with the human spinal cord. Indeed, given the comprehensive representation of the spinal cord periphery with circumferentially implanted interfaces, it is anticipated that a larger spinal cord dimension will allow the design of arrays with greater electrode density to better enhance the degree of selectivity in stimulation [33] and recording.

Accompanied by advances in biomaterials and signal processing, neuroprosthetics according to embodiments of the present invention can potentially restore volitional motor function to SCI patients and act as a key tool during rehabilitation or prognostics, especially for those suffering from an incomplete injury.

The investigations and tests described above have demonstrated that trigger signals for targeted spinal cord stimulation can be generated by direct spinal cord recordings using a low-latency electronic bypass, restoring hindlimb motion in acute spinal cord injury. This was made possible by the circumferential implantation of a thin, flexible device with high electrode density to allow comprehensive representation and stimulation of the spinal cord. Thus direct interfacing with the spinal cord represents a viable strategy for bypassing a site of injury in the spinal cord, restoring volitional motor function to SCI patients.

*Alternative Configurations*

The above description has focused on devices which sense neural signals from a patient's spinal cord at one location and which stimulate tracks in the patient's spinal cord at another location. However, the general principles of bypassing the site of a SCI can be applied using devices located in different parts of the patient's anatomy such as directly on individual nerves.

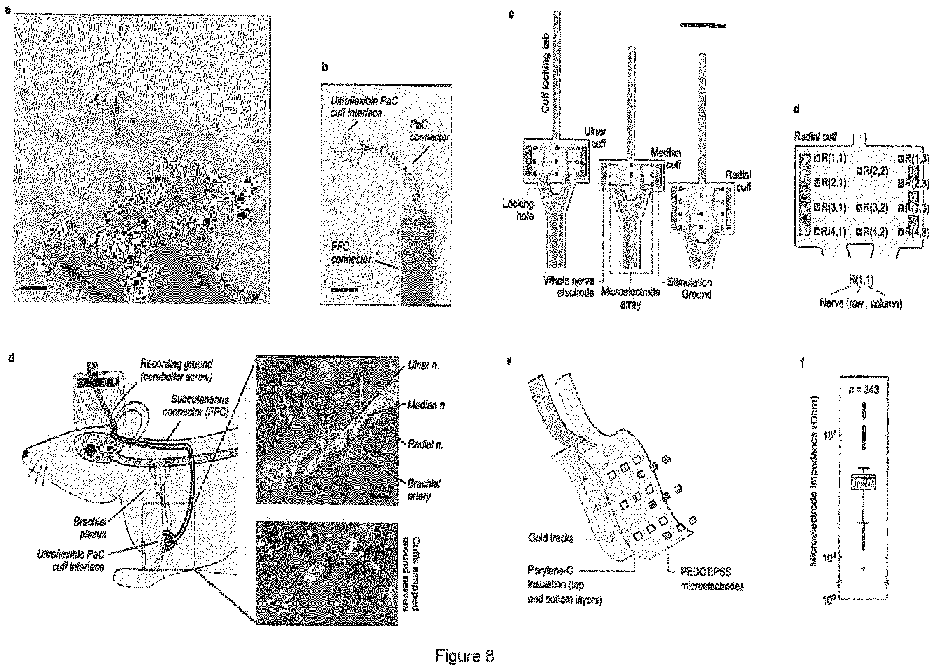

Figure 8 illustrates devices that may be used in alternative embodiments and implanted at different anatomical locations such as nerves.

Figure 8a is a photograph of a flexible nerve cuff array device on a cotton ball, highlighting its flexible and lightweight construction.

Figure 8b is a photograph of the flexible nerve cuff array device. The cuffs are fabricated from parylene-C (PaC). These carry electrode connections through a PaC connector, bonded to a flat flexible cable (FFC) connector through which they are externalised. The shape of the PaC connector is designed to accommodate the shape and movement of the rat shoulder, and contains multiple tabs to facilitate surgical manipulation. The skilled person will appreciate that variations to the design may be made for implantation at other sites or in other animals.

Figure 8c is a diagram showing the cuff and multielectrode array layout. Each implantable device consists of three cuffs designed for implantation into three individual nerves (Ulnar, Median, and Radial nerves). Each cuff has a regular array of 100 by 100 $\mu$m microelectrodes which are wrapped around the circumference of the nerve during implantation. To seal each cuff, its locking tab is fed through the cuff's locking hole. In addition to the microelectrodes, each cuff contains a larger electrode which can be used for whole nerve stimulation/recording and a ground use for nerve stimulation.

Figure 8d is a diagram displaying a naming convention of microelectrodes (radial nerve cuff shown). It will be noted that the radial nerve cuff lacks a R(1,2) microelectrode due to device channel number limitations.

Figure 8e shows, diagrammatically, a chronic implantation model and images of device implantation into rat brachial plexus nerves. The microelectrode connections of the implanted cuffs are externalised via a subcutaneously-positioned FFC, exiting the rat of the body through a headcap port. For recording, a ground is implanted into the CSF above the rat cerebellum.

Figure 8f shows, schematically, the construction of flexible nerve cuff array devices. The device has gold tracks encapsulated between two layers of 4$\mu$m PaC. The tracks connect to PEDOT:PSS microelectrodes.

Figure 8g shows the impedance of device microelectrodes following fabrication (n = 343 microelectrodes, N = 12 devices). Scale bar a-b: 10 mm, c: 2 mm.

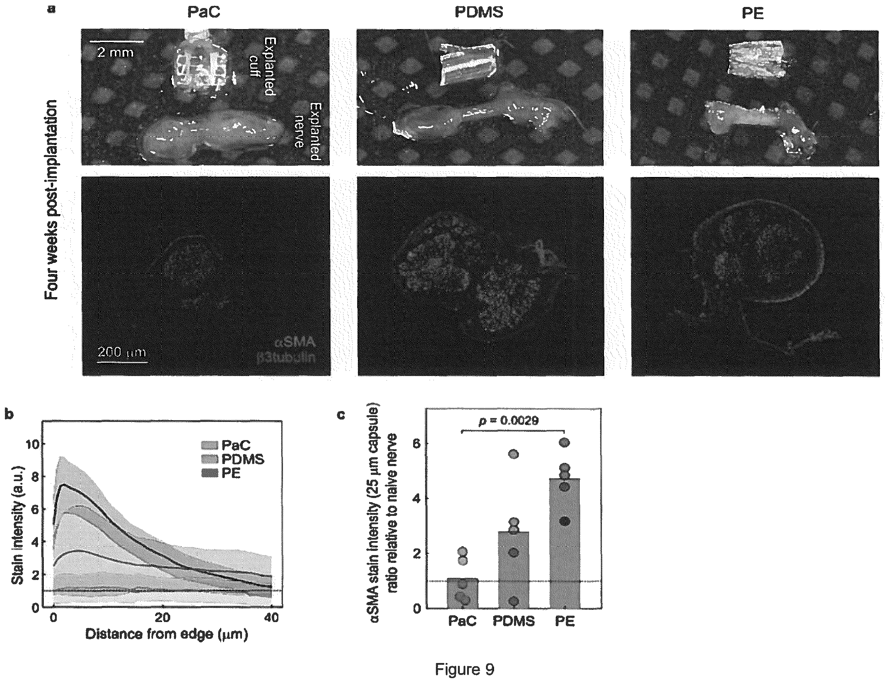

Figure 9 shows foreign body reaction analysis of the flexible PaC nerve cuffs described and illustrated in Figure 8, and shows that these exhibit minimal fibrosis following four weeks of implantation.

Figure 9a is a collection of photographs and immunohistochemistry nerve cross-section images of explanted cuffs and nerves four weeks after implantation. Nerves implanted with ultraflexible PaC cuffs exhibit little fibrosis ($\alpha$SMA) when compared to stiffer polyethylene (PE) cuffs but also soft silicone (PDMS) cuffs.

Figure 9b provides a quantification of fibrosis ($\alpha$SMA stain) at varying depths along the nerve circumference.

Distance from edge of 0 represents tissue in direct contact with the cuff wall. Stain intensity is presented as a ratio of stain relative to non-fibrosed tissue at the centre of the nerve. The flexible PaC cuffs cause lower fibrosis than both PE and PDMS cuffs and almost no fibrosis above background levels. Lines represent group mean and shaded areas standard deviation. N = 5 rats.

Figure 9c provides a quantification of fibrosis ($\alpha$SMA stain) at the 25 $\mu$m of nerve tissue closest to the cuffs. The flexible PaC cuffs cause a significantly lower degree of fibrosis than PE cuffs (p = 0.0029, ANOVA and Tukey post-hoc test). Bars represent the group mean and circles represent values for individual animals.

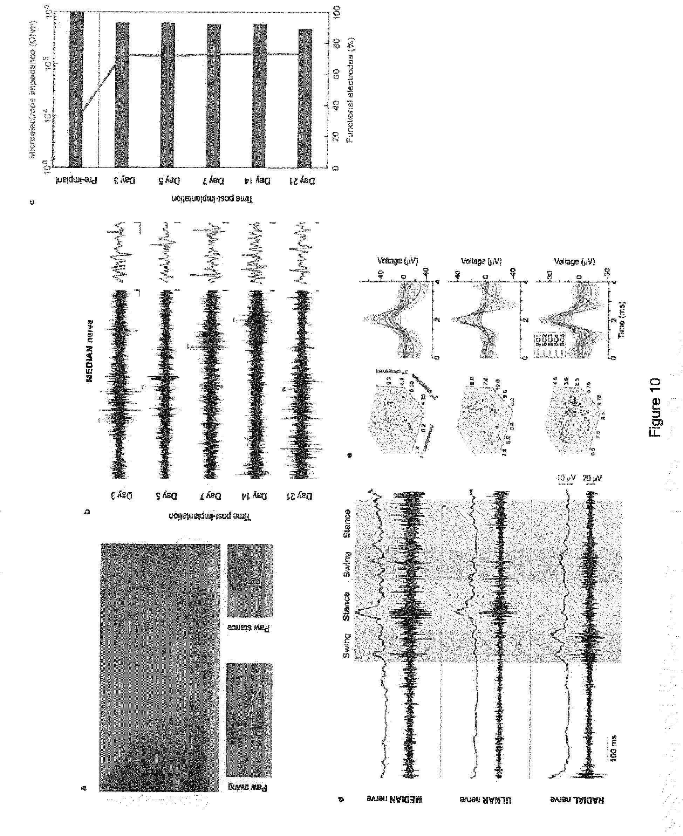

Figure 10 shows that the flexible nerve cuff arrays can achieve stable recordings from their target nerves for long periods of time.

Figure 10a is a series of photographs of behaviour tasks performed by animals during electrophysiology recordings. Animals are recorded while walking down a transparent tunnel. This allows the identification of nerve activity related to the swing and stance phases of walking of the implanted limb.

Figure 10b shows representative traces of recorded activity from the median cuff of a rat over 21 days of implantation. Individual spikes can be seen throughout all timepoints (right, magnified from sections marked by red lines in traces on left). Scale bars: 100 ms and 10 $\mu$V (left) and 5 ms and 10 $\mu$V (right).

Figure 10c shows microelectrode impedance and survival over the implantation period. Implantation leads to an impedance rise and the breakage of 7% of the microelectrodes, but these values remain thereafter stable over the 21 day implantation period. Functional microelectrodes are defined as having an impedance < 1 MOhm. n = 96 (pre-implantation), 89 (days 3-7), 88 (day 14) and 87 (day 21) microelectrodes, N = 3 rats. Experiment was ad hoc designed to terminate at 21 days. One rat was terminated 14 days post-implantation due to skin damage caused by the device FFC connector.

Figure 10d shows representative neural traces (black) and averaged RMS values recorded from median, ulnar, and radial nerves simultaneously, 3 days post-implantation. Neural activity is seen to increase during the implanted limb stance phase of gait in median and ulnar nerves, and during the swing phase in radial nerve.

Figure 10e shows the results of spike sorting performed over the traces in Figure 10d.

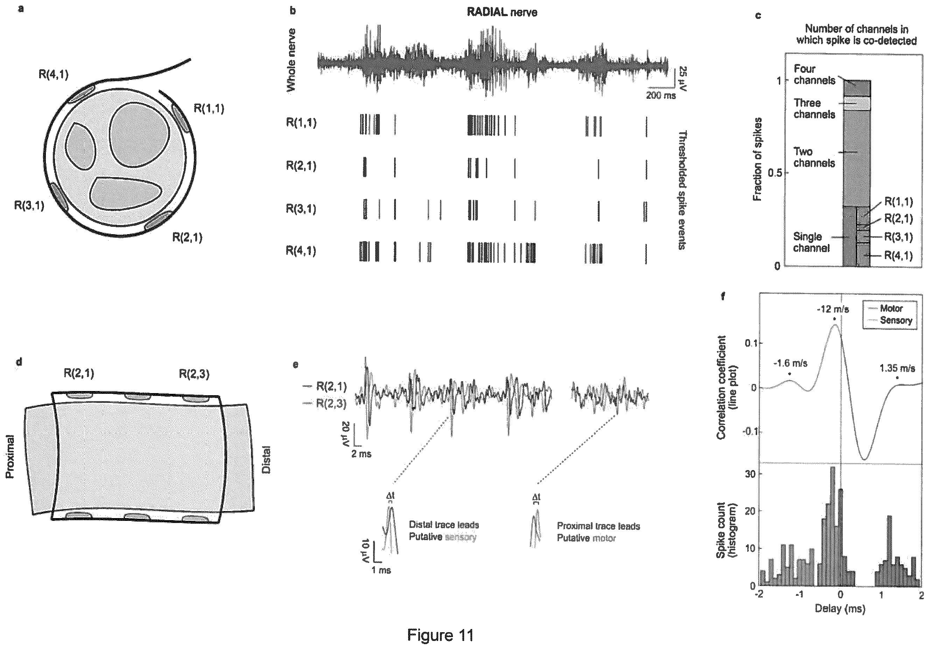

Figure 11 shows that the flexible cuff arrays can achieve sub-nerve recording resolution without penetrating the nerve.

Figure 11a is a diagram of the layout of one column of microelectrodes around the circumference of the radial nerve.

Figure 11b shows spike events across four microelectrodes within a column along the radial nerve circumference. A nerve recording is provided (top) for reference. Spikes are detected in bursts in all microelectrodes, coinciding the swing gait phase during rat walking. Some spikes are detected across all microelectrodes, while others are detected uniquely in some of them.

Figure 11c is a quantification of spike coincidence across microelectrodes for activity shown in Figure 11b. Most neural spikes are detected across two microelectrodes simultaneously, or uniquely by a single microelectrode. All four microelectrodes record these unique spikes (breakdown of proportion represented in graph). Co-detection is quantified by number of spikes, not by co-detection events (an event where four spikes are co-detected across all four microelectrodes is counted as four spikes).

Figure 11d is a diagram of a layout of microelectrodes positioned along the length of the radial nerve.

Figure 11e shows neural activity recording from two columns of microelectrodes. Neural action potential spikes recorded across both rings exhibit a time delay, corresponding to their direction and velocity. This is illustrated at the bottom of the panel.

Figure 11f shows quantifications of radial nerve activity delay between microelectrodes within the most proximal and most distal columns in the array of the cuff over a 2.7 second recording. Top: quantification corresponding to the cross-correlation value between the nerve recordings of the two microelectrode columns at varying time delays. Bottom: histogram corresponding to the inter-spike interval between microelectrode columns. Both analyses identify peaks of activity at delays corresponding to ~1.6 m/s afferent, ~12 m/s afferent, and 1.35 m/s efferent, calculated based on the 2 mm distance between microelectrode rings. Fast afferent/efferent activity is also detected at close to 0 ms delay.

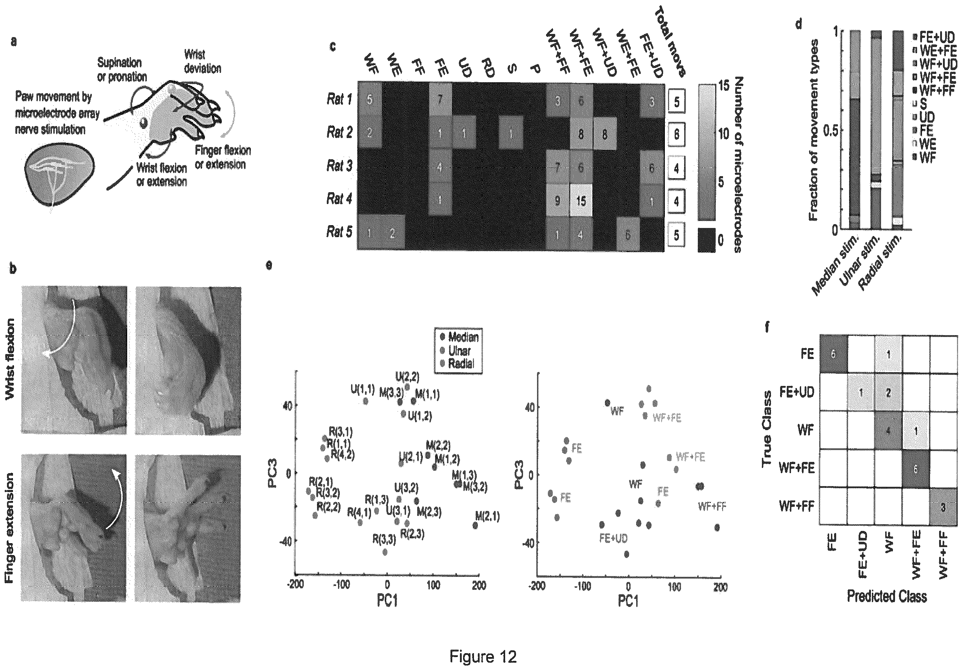

Figure 12 shows that the flexible cuff arrays can achieve sub-nerve stimulation resolution without penetrating the nerve.

Figure 12a is a diagram of the experimental setup. Stimulation of the three implanted nerves using individual microelectrodes causes movements of the wrist and fingers of the paw, in rats under anaesthesia.

Figure 12b is a number of pictures of examples of movements produced before (left) and during (right) nerve stimulation.

Figure 12c is a heatmap of quantification of movements produced by microelectrode stimulation across five rats. While stimulation on occasion caused simple movements, more often combination (containing "+" in name) movements were observed. Certain simple movements were never observed. Despite variations in movements across different implantations and animals, more than three movements were observed in every occasion, indicating higher than whole-nerve stimulation resolution.

Figure 12d is a quantification of the types of movements produced by stimulation using microelectrodes on the three different nerves.

Figure 12e is a plot of the principal components of kinematic analysis of the various movements produced, for Rat 1. Left: movements are tagged based on the microelectrode that produced them. Clustering is seen across groups of microelectrodes within each cuff. Right: movements are tagged by the identified type of movement produced. Clustering indicates that many identified types of movements can be performed with different kinematics, indicating additional stimulation selectivity not captured in Figure 12c.

Figure 12f is a truth matrix for Rat 1 movements classified using a k-nearest neighbour classifier. WF: wrist flexion, WE: wrist extension, FE: finger extension, UD: ulnar deviation, SD: sacral deviation, S: supination, P: pronation, WF+FF: wrist flexion and finger flexion, WF+FE: wrist flexion and finger extension, WF+UD: wrist flexion and ulnar deviation, WE+FE: wrist extension and finger extension, FE+UD: finger extension and ulnar deviation, Total movs: total number of different movements produced.

*System Configuration*

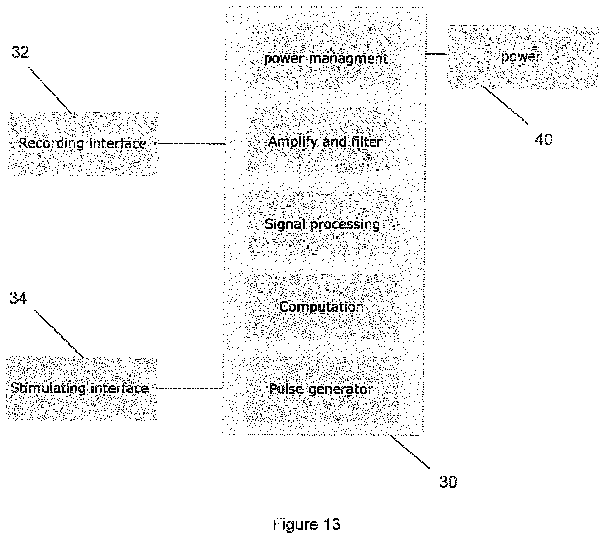

The functional components of a system according to an embodiment of the present invention are shown in Figure 13. The system includes a recording interface 32 and a stimulating interface 34. These interfaces may be provided, for example, by implantable devices 10a, 10b having an electrode array, such as those described previously.

The interfaces 32, 34 are connected to a controller 30 which may comprise one or more processors adapted to run the various processes and methods set out herein and one or more non-volatile memories arranged to store code to cause the processor(s) to run those processes and methods and/or to store data arising from the operation of the system.

The controller 30 can have modules which perform specific tasks such as signal amplification, signal filtering, signal processing, computation, interpretation, pulse generation, etc.. Such modules may be provided by separate processors or combined in any combination in the operation of one or more processors. The controller 30 also has a power management module which is arranged to supply power to the controller itself and to the interfaces 32, 34. The power management module connects to a power supply unit 40.

In certain embodiments, the power supply unit 40 may be a battery. In other embodiments the power supply unit 40 is a wireless power receiver, arranged to receive power wirelessly from an external source. Such an arrangement allows the controller 30, interfaces 32, 34 and power supply unit 40 to be implanted in a patient, and receive power from a source external to the patient, thus removing the need for replacement of batteries. In other embodiments the power supply unit 40 provides a direct wired connection to a power source.

The forgoing description is exemplary in nature only, and the skilled person will understand that changes and variations on the disclosed embodiments are possible within the scope of the claims. The claims define the invention.

**REFERENCES**

1. Wagner, F. B. et al. Targeted neurotechnology restores walking in humans with spinal cord injury. Nature 563, 65-71 (2018).

2. Angeli, C. A. et al. Recovery of Over-Ground Walking after Chronic Motor Complete Spinal Cord Injury. N Engl J Med 379, 1244-1250 (2018).

3. Gill, M. L. et al. Neuromodulation of lumbosacral spinal networks enables independent stepping after complete paraplegia. Nat Med 24, 1677-1682 (2018).

4. Capogrosso, M. et al. A brain-spine interface alleviating gait deficits after spinal cord injury in primates. Nature 539, 284-288 (2016).

5. Formento, E. et al. Electrical spinal cord stimulation must preserve proprioception to enable locomotion in humans with spinal cord injury. Nat Neurosci 21, 1728-1741 (2018).

6. Sharpe, A. N. & Jackson, A. Upper-limb muscle responses to epidural, subdural and intraspinal stimulation of the cervical spinal cord. J. Neural Eng. 11, 016005 (2014).

7. Hogan, M. K. et al. A wireless spinal stimulation system for ventral activation of the rat cervical spinal cord. Sci Rep 11, 14900 (2021).

8. Guiho, T., Baker, S. N. & Jackson, A. Epidural and transcutaneous spinal cord stimulation facilitates descending inputs to upper-limb motoneurons in monkeys. J. Neural Eng. 18, 046011 (2021).

9. Prasad, A. & Sahin, M. Can motor volition be extracted from the spinal cord? J NeuroEngineeringRehabil 9, 41 (2012).

10. Fathi, Y. & Erfanian, A. Decoding hindlimb kinematics from descending and ascending neural signals during cat locomotion. J Neural Eng (2021) doi:10.1088/1741-2552/abd82a.

11. Song, E., Li, J., Won, S. M., Bai, W. & Rogers, J. A. Materials for flexible bioelectronic systems as chronic neural interfaces. Nature Materials 19, 590-603 (2020).

12. Woodington, B. J. et al. Electronics with shape actuation for minimally invasive spinal cord stimulation. Science Advances 7, eabg7833 (2021).

13. Nelson, M. J., Valtcheva, S. & Venance, L. Magnitude and behavior of cross-talk effects in multichannel electrophysiology experiments. J Neurophysiol 118, 574-594 (2017).

14. Khodagholy, D. et al. In vivo recordings of brain activity using organic transistors. Nature Communications 4, 1575 (2013).

15. Castagnola, V. et al. Parylene-based flexible neural probes with PEDOT coated surface for brain stimulation and recording. Biosensors and Bioelectronics 67, 450-457 (2015).

16. Thirumala, P. et al. Diagnostic accuracy of evoked potentials for functional impairment after contusive spinal cord injury in adult rats. J Clin Neurosci 25, 122-126 (2016).

17. Volkov, A. V. et al. Understanding the Capacitance of PEDOT:PSS. Advanced Functional Materials 27, 1700329 (2017).

18. Jiang, L., Woodington, B., Carnicer-Lombarte, A., Malliaras, G. & Barone, D. G. Spinal cord bioelectronic interfaces: opportunities in neural recording and clinical challenges. J. Neural Eng. 19, 021003 (2022).

19. Webb, A. A. & Muir, G. D. Unilateral dorsal column and rubrospinal tract injuries affect overground locomotion in the unrestrained rat. European Journal of Neuroscience 18, 412-422 (2003).

20. Raineteau, O., Fouad, K., Bareyre, F. M. & Schwab, M. E. Reorganization of descending motor tracts in the rat spinal cord. Eur J Neurosci 16, 1761-1771 (2002).

21. Diedrich, A., Charoensuk, W., Brychta, R. J., Ertl, A. C. & Shiavi, R. Analysis of Raw Microneurographic Recordings Based on Wavelet De-Noising Technique and Classification Algorithm: Wavelet Analysis in Microneurography. IEEE Trans Biomed Eng 50, 41-50 (2003).

22. Strahm, C., Min, K., Boos, N., Ruetsch, Y. & Curt, A. Reliability of perioperative SSEP recordings in spine surgery. Spinal Cord 41, 483-489 (2003).

23. Parker, J. L., Shariati, N. H. & Karantonis, D. M. Electrically evoked compound action potential recording in peripheral nerves. Bioelectronics in Medicine 1, 71-83 (2018).

24. Carnicer-Lombarte, A., Chen, S.-T., Malliaras, G. G. & Barone, D. G. Foreign Body Reaction to Implanted Biomaterials and Its Impact in Nerve Neuroprosthetics. Front. Bioeng. Biotechnol. 9, (2021).

25. Prasad, A. & Sahin, M. Characterization of neural activity recorded from the descending tracts of the rat spinal cord. Front. Neurosci. 4, (2010).

26. Streeter, K. A. et al. Coupling multielectrode array recordings with silver labeling of recording sites to study cervical spinal network connectivity. J Neurophysiol 117, 1014-1029 (2017).

27. Barrese, J. C., Aceros, J. & Donoghue, J. P. Scanning electron microscopy of chronically implanted intracortical microelectrode arrays in non-human primates. J Neural Eng 13, 026003 (2016).

28. Noga, B. et al. Field potential mapping of neurons in the lumbar spinal cord activated following stimulation of the mesencephalic locomotor region. J. Neurosci. 15, 2203-2217 (1995).

29. Ichiyama, R. M., Gerasimenko, Y. P., Zhong, H., Roy, R. R. & Edgerton, V. R. Hindlimb stepping movements in complete spinal rats induced by epidural spinal cord stimulation. Neurosci Lett 383, 339-344 (2005).

30. Ibáñez, J. et al. Low Latency Estimation of Motor Intentions to Assist Reaching Movements along Multiple Sessions in Chronic Stroke Patients: A Feasibility Study. Frontiers in Neuroscience 11, 126 (2017).

31. Lacour, S. P., Courtine, G. & Guck, J. Materials and technologies for soft implantable neuroprostheses. Nature Reviews Materials 1, 1-14 (2016).

32. Saliani, A. et al. Axon and Myelin Morphology in Animal and Human Spinal Cord. Frontiers in Neuroanatomy 11, 129 (2017).

33. Ryynänen, O. R. M., Hyttinen, J. A. K., Laarne, P. H. & Malmivuo, J. A. Effect of electrode density and measurement noise on the spatial resolution of cortical potential distribution. IEEE Trans Biomed Eng 51, 1547-1554 (2004).

34. Rivnay, J. et al. Structural control of mixed ionic and electronic transport in conducting polymers. Nat Commun 7, 11287 (2016).

35. Sorrenti, V. et al. Understanding the Effects of Anesthesia on Cortical Electrophysiological Recordings: A Scoping Review. Int J Mol Sci 22, (2021).

36. Mostany, R. & Portera-Cailliau, C. A Craniotomy Surgery Procedure for Chronic Brain Imaging. J Vis Exp 680 (2008) doi:10.3791/680.

37. Keast, J. R., Forrest, S. L. & Osborne, P. B. Sciatic nerve injury in adult rats causes distinct changes in the central projections of sensory neurons expressing different glial cell line-derived neurotrophic factor family receptors. J Comp Neurol 518, 3024-3045 (2010).

38. Batt, J. A. E. & Bain, J. R. Tibial Nerve Transection - A Standardized Model for Denervation-induced Skeletal Muscle Atrophy in Mice. J Vis Exp 50657 (2013) doi:10.3791/50657.

39. Shah, S. B. et al. Does cryoneurolysis result in persistent motor deficits? A controlled study using a rat peroneal nerve injury model. Reg Anesth Pain Med 45, 287-292 (2020).

40. Gelderd, J. B. & Chopin, S. F. The vertebral level of origin of spinal nerves in the rat. Anat Rec 188, 45-47 (1977).

41. Pelot, N. A., Thio, B. J. & Grill, W. M. Modeling Current Sources for Neural Stimulation in COMSOL. Frontiers in Computational Neuroscience 12, (2018).

42. Brown, A. R. & Martinez, M. Ipsilesional Motor Cortex Plasticity Participates in Spontaneous Hindlimb Recovery after Lateral Hemisection of the Thoracic Spinal Cord in the Rat. J. Neurosci. 38, 9977-9988 (2018).

43. Watson, M., Sawan, M. & Dancause, N. The Duration of Motor Responses Evoked with Intracortical Microstimulation in Rats Is Primarily Modulated by Stimulus Amplitude and Train Duration. PLOS ONE 11, e0159441 (2016).

44. Fehlings, M. G., Tator, C. H., Linden, R. D. & Piper, 1. R. Motor evoked potentials recorded from normal and spinal cord-injured rats. Neurosurgery 20, 125-130 (1987).

45. Donoho, D. L. & Johnstone, I. M. Ideal Spatial Adaptation by Wavelet Shrinkage. Biometrika 81, 425-455 (1994).

46. Campbell, E., Phinyomark, A. & Scheme, E. Feature Extraction and Selection for Pain Recognition Using Peripheral Physiological Signals. Frontiers in Neuroscience 13, 437 (2019).

47. Multivariate Statistical Data Analysis- Principal Component Analysis (PCA). International Journal of Livestock Research http://ijlr.org/issue/multivariate-statistical-data-analysis-principal-component-analysis-pca/.

48. Jolliffe, I. T. & Cadima, J. Principal component analysis: a review and recent developments. Philosophical Transactions of the Royal Society A: Mathematical, Physical and Engineering Sciences 374, 20150202 (2016).

49. McInnes, L., Healy, J. & Melville, J. UMAP: Uniform Manifold Approximation and Projection for Dimension Reduction. arXiv:1802.03426 [cs, stat] (2020).

50. Pestov, V. Is the k-NN classifier in high dimensions affected by the curse of dimensionality? Computers & Mathematics with Applications 65, 1427-1437 (2013).

51. Li, M., Xu, H., Liu, X. & Lu, S. Emotion recognition from multichannel EEG signals using K-nearest neighbor classification. Technol Health Care 26, 509-519.

52. Mathis, A. et al. DeepLabCut: markerless pose estimation of user-defined body parts with deep learning. Nat Neurosci 21, 1281-1289 (2018).

All of the above references are hereby incorporated by reference in their entirety.

**Claims**

1. A system for treating a patient having a spinal cord injury, the system including:

   a first flexible implantable device and having a first plurality of electrodes;
   a second flexible implantable device and having a second plurality of electrodes; and
   a controller communicatively coupled to the electrodes of the first and second implantable devices and arranged to receive inputs from the electrodes of the first implantable device, process said inputs to produce stimulation signals and send said stimulation signals to the electrodes of the second implantable device.

2. A system according to claim 1 wherein either or both of the first and second implantable devices are configured to conform to the spinal cord or a nerve of the patient when implanted in the patient..

3. A system according to claim 2 wherein either or both of the first and second implantable devices have a gel layer arranged on a surface of the device opposed to a surface of the device having the plurality of electrodes.

4. A system according to any one of the preceding claims wherein either or both of the first and second implantable devices are configured to substantially encircle the spinal cord or nerve of the patient when implanted in the patient.

5. A system according to any one of the preceding claims wherein the first and second implantable devices each have a distal portion and each distal portion contains the respective plurality of electrodes, further wherein the distal portions of the first and second implantable devices are identical.

6. A system according to any one of claims 1 to 4 wherein the first implantable device is configured for implantation in the brain of the patient.

7. A system according to any of claims 1 to 4 or 6 wherein the second implantable device is configured for implantation in a limb of the patient.

8. A system according to any one of the preceding claims wherein the controller is arranged to:

   process the inputs by decoding the inputs to determine an intended stimulation of nerves of the patient rostral of the position of the first device on the spinal cord; and
   produce stimulation signals that, when applied to the patient's spinal cord by the second device, produce said intended stimulation of the nerves of the patient.

9. A system according to any one of the preceding claims wherein the controller is arranged to process said inputs by integrating the inputs in both the time and spatial domains.

10. A system according to claim 9 wherein the controller is further arranged to identify unique topographical signatures from the integrated inputs to identify the dominant spinal cord tract which was activated.

11. A method of treating a patient having a spinal cord injury, the method including the steps of:

percutaneously implanting a first plurality of electrodes to a position upstream of the location of the spinal cord injury;
percutaneously implanting a second plurality of electrodes to a position downstream of the location of the spinal cord injury;
processing signals from one or more of the first plurality of electrodes to determine an intended stimulation of nerves of the patient downstream of the position of the spinal cord injury;
producing stimulation signals that, when applied to the patient's nerves through one or more of the second plurality of electrodes, produce said intended stimulation of the nerves of the patient.

12. A method according to claim 11 wherein either or both of the first plurality of electrodes and the second plurality of electrodes are implanted adjacent to the spinal cord or a nerve of the patient.

13. A method according to claim 12 wherein either or both of the first plurality of electrodes and the second plurality of electrodes are implanted such that they substantially encircle the spinal cord or a nerve of the patient.

14. A method according to any one of claims 11 to 13 wherein the first plurality of electrodes are implanted in the brain of the patient.

15. A method according to any one of claims 11 to 14 wherein the second plurality of electrodes are implanted in a limb of the patient.

Figure 1

Figure 2

a) Clean glass or Si wafter

b) CVD parylene-c

c) Spin coat photoresist

d) UV photolithography

e) Metal deposition

f) Lift off

g) CVD parylene-c

h) Spin coat photoresist

i) UV photolithography

j) RIE outline

k) CVD parylene-c

l) UV photolithography

m) RIE and Spin coat PEDOT:PSSc

n) Peel back PEDOT:PSS

o) Soak device and release

p) ACF bond FFC

Figure 3

Figure 4

Figure 5

Figure 6

Figure 7

Figure 8

Figure 9

Figure 10

Figure 11

EP 4 368 240 A1

Figure 12

32

Recording interface

34

Stimulating interface

power managment

power

40

Amplify and filter

Signal processing

Computation

Pulse generator

30

Figure 13

Europäisches
Patentamt

European
Patent Office

Office européen
des brevets

# EUROPEAN SEARCH REPORT

**Application Number**

EP 22 38 6079

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JIANG LEI ET AL: "Spinal cord bioelectronic interfaces: opportunities in neural recording and clinical challenges", JOURNAL OF NEURAL ENGINEERING, IOP PUBLISHING, BRISTOL, GB, vol. 19, no. 2, 14 April 2022 (2022-04-14), XP020420617, ISSN: 1741-2560, DOI: 10.1088/1741-2552/AC605F [retrieved on 2022-04-14] | 1-8, 11-15 | INV. A61N1/05 A61N1/36 ADD. A61N1/378 |
| A | * Abstract, Sections 6.9 and 7; figure 5 * | 9,10 | |
| X | US 2012/123293 A1 (SHAH JAWAD A [US] ET AL) 17 May 2012 (2012-05-17) * paragraphs [0018], [0019], [0036], [0060] * | 1,4,6-8 | |
| A | ANNETTA NICHOLAS V ET AL: "A High Definition Noninvasive Neuromuscular Electrical Stimulation System for Cortical Control of Combinatorial Rotary Hand Movements in a Human With Tetraplegia", IEEE TRANSACTIONS ON BIOMEDICAL ENGINEERING, IEEE, USA, vol. 66, no. 4, 1 April 2019 (2019-04-01), pages 910-919, XP011715412, ISSN: 0018-9294, DOI: 10.1109/TBME.2018.2864104 [retrieved on 2019-03-18] * the whole document * | 1,11 | TECHNICAL FIELDS SEARCHED (IPC) A61N |
| A | US 2021/038887 A1 (BOUTON CHAD E [US] ET AL) 11 February 2021 (2021-02-11) * the whole document * | 1,11 | |

The present search report has been drawn up for all claims

| Place of search | Date of completion of the search | Examiner |
|---|---|---|
| Munich | 31 March 2023 | Aronsson, Fredrik |

## EP 4 368 240 A1

### ANNEX TO THE EUROPEAN SEARCH REPORT
### ON EUROPEAN PATENT APPLICATION NO.

EP 22 38 6079

31-03-2023

| Patent document cited in search report | | Publication date | Patent family member(s) | | Publication date |
|---|---|---|---|---|---|
| US 2012123293 | A1 | 17-05-2012 | CA | 2854258 A1 | 18-05-2012 |
| | | | US | 2012123293 A1 | 17-05-2012 |
| | | | WO | 2012064968 A1 | 18-05-2012 |
| US 2021038887 | A1 | 11-02-2021 | AU | 2016270928 A1 | 21-12-2017 |
| | | | AU | 2020277086 A1 | 17-12-2020 |
| | | | EP | 3302688 A1 | 11-04-2018 |
| | | | EP | 3782696 A1 | 24-02-2021 |
| | | | EP | 4137196 A1 | 22-02-2023 |
| | | | US | 2018178008 A1 | 28-06-2018 |
| | | | US | 2021038887 A1 | 11-02-2021 |
| | | | WO | 2016196797 A1 | 08-12-2016 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WAGNER, F. B. et al.** Targeted neurotechnology restores walking in humans with spinal cord injury. *Nature,* 2018, vol. 563, 65-71
- **ANGELI, C. A. et al.** Recovery of Over-Ground Walking after Chronic Motor Complete Spinal Cord Injury. *N Engl J Med,* 2018, vol. 379, 1244-1250
- **GILL, M. L. et al.** Neuromodulation of lumbosacral spinal networks enables independent stepping after complete paraplegia. *Nat Med,* 2018, vol. 24, 1677-1682
- **CAPOGROSSO, M. et al.** A brain-spine interface alleviating gait deficits after spinal cord injury in primates. *Nature,* 2016, vol. 539, 284-288
- **FORMENTO, E. et al.** Electrical spinal cord stimulation must preserve proprioception to enable locomotion in humans with spinal cord injury. *Nat Neurosci,* 2018, vol. 21, 1728-1741
- **SHARPE, A. N. ; JACKSON, A.** Upper-limb muscle responses to epidural, subdural and intraspinal stimulation of the cervical spinal cord. *J. Neural Eng.,* 2014, vol. 11, 016005
- **HOGAN, M. K. et al.** A wireless spinal stimulation system for ventral activation of the rat cervical spinal cord. *Sci Rep,* 2021, vol. 11, 14900
- **GUIHO, T. ; BAKER, S. N. ; JACKSON, A.** Epidural and transcutaneous spinal cord stimulation facilitates descending inputs to upper-limb motoneurons in monkeys. *J. Neural Eng.,* 2021, vol. 18, 046011
- **PRASAD, A. ; SAHIN, M.** Can motor volition be extracted from the spinal cord?. *J NeuroEngineering Rehabil,* 2012, vol. 9, 41
- **FATHI, Y. ; ERFANIAN, A.** Decoding hindlimb kinematics from descending and ascending neural signals during cat locomotion. *J Neural Eng,* 2021
- **SONG, E. ; LI, J. ; WON, S. M. ; BAI, W. ; ROGERS, J. A.** Materials for flexible bioelectronic systems as chronic neural interfaces. *Nature Materials,* 2020, vol. 19, 590-603
- **WOODINGTON, B. J. et al.** Electronics with shape actuation for minimally invasive spinal cord stimulation. *Science Advances,* 2021, vol. 7, eabg7833
- **NELSON, M. J. ; VALTCHEVA, S. ; VENANCE, L.** Magnitude and behavior of cross-talk effects in multichannel electrophysiology experiment. *J Neurophysiol,* 2017, vol. 118, 574-594
- **KHODAGHOLY, D. et al.** In vivo recordings of brain activity using organic transistors. *Nature Communications,* 2013, vol. 4, 1575
- **CASTAGNOLA, V. et al.** Parylene-based flexible neural probes with PEDOT coated surface for brain stimulation and recording. *Biosensors and Bioelectronics,* 2015, vol. 67, 450-457
- **THIRUMALA, P. et al.** Diagnostic accuracy of evoked potentials for functional impairment after contusive spinal cord injury in adult rats. *J Clin Neurosci,* 2016, vol. 25, 122-126
- **VOLKOV, A. V. et al.** Understanding the Capacitance of PEDOT:PSS. *Advanced Functional Materials,* 2017, vol. 27, 1700329
- **JIANG, L. ; WOODINGTON, B. ; CARNICER-LOMBARTE, A. ; MALLIARAS, G. ; BARONE, D. G.** Spinal cord bioelectronic interfaces: opportunities in neural recording and clinical challenges. *J. Neural Eng.,* 2022, vol. 19, 021003
- **WEBB, A. A. ; MUIR, G. D.** Unilateral dorsal column and rubrospinal tract injuries affect overground locomotion in the unrestrained rat. *European Journal of Neuroscience,* 2003, vol. 18, 412-422
- **RAINETEAU, O. ; FOUAD, K. ; BAREYRE, F. M. ; SCHWAB, M. E.** Reorganization of descending motor tracts in the rat spinal cord. *Eur J Neurosci,* 2002, vol. 16, 1761-1771
- **DIEDRICH, A. ; CHAROENSUK, W. ; BRYCHTA, R. J. ; ERTL, A. C. ; SHIAVI, R.** Analysis of Raw Microneurographic Recordings Based on Wavelet De-Noising Technique and Classification Algorithm: Wavelet Analysis in Microneurography. *IEEE Trans Biomed Eng,* 2003, vol. 50, 41-50
- **STRAHM, C. ; MIN, K. ; BOOS, N. ; RUETSCH, Y. ; CURT, A.** Reliability of perioperative SSEP recordings in spine surgery. *Spinal Cord,* 2003, vol. 41, 483-489
- **PARKER, J. L. ; SHARIATI, N. H. ; KARANTONIS, D. M.** Electrically evoked compound action potential recording in peripheral nerves. *Bioelectronics in Medicine,* 2018, vol. 1, 71-83
- **CARNICER-LOMBARTE, A. ; CHEN, S.-T. ; MALLIARAS, G. G. ; BARONE, D. G.** Foreign Body Reaction to Implanted Biomaterials and Its Impact in Nerve Neuroprosthetics. *Front. Bioeng. Biotechnol.,* 2021, vol. 9
- **PRASAD, A. ; SAHIN, M.** Characterization of neural activity recorded from the descending tracts of the rat spinal cord. *Front. Neurosci.,* 2010, vol. 4

- **STREETER, K. A. et al.** Coupling multielectrode array recordings with silver labeling of recording sites to study cervical spinal network connectivity. *J Neurophysiol,* 2017, vol. 117, 1014-1029
- **BARRESE, J. C. ; ACEROS, J. ; DONOGHUE, J. P.** Scanning electron microscopy of chronically implanted intracortical microelectrode arrays in non-human primate. *J Neural Eng,* 2016, vol. 13, 026003
- **NOGA, B. et al.** Field potential mapping of neurons in the lumbar spinal cord activated following stimulation of the mesencephalic locomotor region. *J. Neurosci.,* 1995, vol. 15, 2203-2217
- **ICHIYAMA, R. M. ; GERASIMENKO, Y. P. ; ZHONG, H. ; ROY, R. R. ; EDGERTON, V. R.** Hindlimb stepping movements in complete spinal rats induced by epidural spinal cord stimulation. *Neurosci Lett,* 2005, vol. 383, 339-344
- **IBÁÑEZ, J. et al.** Low Latency Estimation of Motor Intentions to Assist Reaching Movements along Multiple Sessions in Chronic Stroke Patients: A Feasibility Study. *Frontiers in Neuroscience,* 2017, vol. 11, 126
- **LACOUR, S. P. ; COURTINE, G. ; GUCK, J.** Materials and technologies for soft implantable neuroprostheses. *Nature Reviews Materials,* 2016, vol. 1, 1-14
- **SALIANI, A. et al.** Axon and Myelin Morphology in Animal and Human Spinal Cord. *Frontiers in Neuroanatomy,* 2017, vol. 11, 129
- **RYYNÄNEN, O. R. M. ; HYTTINEN, J. A. K. ; LAARNE, P. H. ; MALMIVUO, J. A.** Effect of electrode density and measurement noise on the spatial resolution of cortical potential distribution. *IEEE Trans Biomed Eng,* 2004, vol. 51, 1547-1554
- **RIVNAY, J. et al.** Structural control of mixed ionic and electronic transport in conducting polymers. *Nat Commun,* 2016, vol. 7, 11287
- **SORRENTI, V. et al.** Understanding the Effects of Anesthesia on Cortical Electrophysiological Recordings: A Scoping Review. *Int J Mol Sci,* 2021, vol. 22
- **MOSTANY, R. ; PORTERA-CAILLIAU, C.** A Craniotomy Surgery Procedure for Chronic Brain Imaging. *J Vis Exp,* 2008, vol. 680
- **KEAST, J. R. ; FORREST, S. L. ; OSBORNE, P. B.** Sciatic nerve injury in adult rats causes distinct changes in the central projections of sensory neurons expressing different glial cell line-derived neurotrophic factor family receptors. *J Comp Neurol,* 2010, vol. 518, 3024-3045
- **BATT, J. A. E. ; BAIN, J. R.** Tibial Nerve Transection - A Standardized Model for Denervation-induced Skeletal Muscle Atrophy in Mice. *J Vis Exp,* 2013, 50657
- **SHAH, S. B. et al.** Does cryoneurolysis result in persistent motor deficits? A controlled study using a rat peroneal nerve injury model. *Reg Anesth Pain Med,* 2020, vol. 45, 287-292
- **GELDERD, J. B. ; CHOPIN, S. F.** The vertebral level of origin of spinal nerves in the rat. *Anat Rec,* 1977, vol. 188, 45-47
- **PELOT, N. A. ; THIO, B. J. ; GRILL, W. M.** Modeling Current Sources for Neural Stimulation in COMSOL. *Frontiers in Computational Neuroscience,* 2018, vol. 12
- **BROWN, A. R. ; MARTINEZ, M.** Ipsilesional Motor Cortex Plasticity Participates in Spontaneous Hindlimb Recovery after Lateral Hemisection of the Thoracic Spinal Cord in the Rat. *J. Neurosci.,* 2018, vol. 38, 9977-9988
- **WATSON, M. ; SAWAN, M. ; DANCAUSE, N.** The Duration of Motor Responses Evoked with Intracortical Microstimulation in Rats Is Primarily Modulated by Stimulus Amplitude and Train Duration. *PLOS ONE,* 2016, vol. 11, e0159441
- **FEHLINGS, M. G. ; TATOR, C. H. ; LINDEN, R. D ; PIPER, 1. R.** Motor evoked potentials recorded from normal and spinal cord-injured rats. *Neurosurgery,* 1987, vol. 20, 125-130
- **DONOHO, D. L. ; JOHNSTONE, I. M.** Ideal Spatial Adaptation by Wavelet Shrinkage. *Biometrika,* 1994, vol. 81, 425-455
- **CAMPBELL, E. ; PHINYOMARK, A. ; SCHEME, E.** Feature Extraction and Selection for Pain Recognition Using Peripheral Physiological Signals. *Frontiers in Neuroscience,* 2019, vol. 13, 437
- Multivariate Statistical Data Analysis- Principal Component Analysis (PCA). *International Journal of Livestock Research, http://ijlr.org/issue/multivariate-statistical-data-analysis-principal-component-analysis-pca*
- **JOLLIFFE, I. T. ; CADIMA, J.** Principal component analysis: a review and recent developments. *Philosophical Transactions of the Royal Society A: Mathematical, Physical and Engineering Sciences,* 2016, vol. 374, 20150202
- **MCINNES, L. ; HEALY, J. ; MELVILLE, J.** UMAP: Uniform Manifold Approximation and Projection for Dimension Reduction. *arXiv:1802.03426,* 2020
- **PESTOV, V.** Is the k-NN classifier in high dimensions affected by the curse of dimensionality?. *Computers & Mathematics with Applications,* 2013, vol. 65, 1427-1437
- **LI, M. ; XU, H. ; LIU, X. ; LU, S.** Emotion recognition from multichannel EEG signals using K-nearest neighbor classification. *Technol Health Care,* vol. 26, 509-519
- **MATHIS, A. et al.** DeepLabCut: markerless pose estimation of user-defined body parts with deep learning. *Nat Neurosci,* 2018, vol. 21, 1281-1289